(19) **Europäisches Patentamt / European Patent Office / Office européen des brevets**

(11) **EP 4 596 691 A1**

(12) **EUROPEAN PATENT APPLICATION**
published in accordance with Art. 153(4) EPC

(43) Date of publication:
06.08.2025 Bulletin 2025/32

(21) Application number: 23873119.4

(22) Date of filing: 26.09.2023

(51) International Patent Classification (IPC):
C12N 15/10 (2006.01)          C12N 15/113 (2010.01)
C12Q 1/6806 (2018.01)         C40B 50/06 (2006.01)

(52) Cooperative Patent Classification (CPC):
C12N 15/10; C12N 15/113; C12Q 1/6806;
C40B 50/06

(86) International application number:
PCT/KR2023/014843

(87) International publication number:
WO 2024/072006 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.09.2022  KR 20220123735
               14.10.2022  KR 20220132561

(71) Applicants:
• POSTECH Research and Business Development
  Foundation
  Pohang-si, Gyeongsangbuk-do 37673 (KR)
• Korea Advanced Institute of Science and
  Technology
  Daejeon 34141 (KR)

(72) Inventors:
• LEE, Jeong Wook
  Pohang-si, Gyeongsangbuk-do 37673 (KR)
• LEE, Jiseon
  Incheon 21584 (KR)
• CHO, Byung Kwan
  Daejeon 34141 (KR)
• JEON, Hahyeon
  Ulsan 44627 (KR)
• CHOE, Dong Hui
  Daejeon 34141 (KR)

(74) Representative: Altmann Stößel Dick
Patentanwälte PartG mbB
Theodor-Heuss-Anlage 2
68165 Mannheim (DE)

Remarks:
The complete document including Reference
Table(s) and the Sequence Listing(s) can be
downloaded from the EPO website

(54) **METHOD FOR GENERATING TOTAL MRNA-BASED RANDOM SGRNA LIBRARY THROUGH ENZYMATIC REACTION**

(57)    The present disclosure relates to a method for generating a total mRNA-based random sgRNA library through an enzymatic reaction. The method according to the present disclosure may generate an sgRNA library that may target the entire genome of any organism, including prokaryotes, without relying on genome sequence information and may be used for phenotype-based screening in prokaryotes because the generated sgRNAs selectively bind only to non-template strands of a target gene. In addition, mRNA, which is a useful genetic material, as well as DNA may be used as a starting material, enabling the generation of an sgRNA library targeting an actively expressed gene.

【Fig. 1a】

**Description**

**[Technical Field]**

The present disclosure relates to a method for generating a total mRNA-based random sgRNA library through an enzymatic reaction.

[Background Art]

Phenotype-based screening is a research method that randomly manipulates the genes of a target organism, such as by deletion or suppression, and then selects and examines cells that show a desired phenotypic change to identify the gene that caused the phenotype change, and is actively performed on various types of organisms, including bacteria, to discover valuable characteristics.

In order to induce phenotype changes in the target organism, the genome must be manipulated on a large scale and with high efficiency, and the CRISPR-Cas9 (clustered regularly interspaced palindromic repeats-Cas9) system has been widely used as an effective genetic manipulation tool.

In particular, when performing phenotype-based screening in prokaryotes, including bacteria, since cells die when genomic DNA is cleaved, genes must be suppressed with GRISPR interference (CRISPRi) utilizing dCas9 with nuclease activity removed. The CRISPRi system requires a single guide RNA (sgRNA) with a sequence complementary to the target DNA to bind to the corresponding DNA, and enables targeting of different regions by simply changing this sequence.

Here, to facilitate phenotype-based screening, sgRNA libraries that target the entire genome of the target organism under study are essential. The traditional method for generating such sgRNA libraries is to directly design sgRNAs based on genomic information and then synthesize the sgRNAs on a microarray basis, which is not only time-consuming and costly, but also impossible to design without high-quality genetic information, making it unsuitable for many organisms that have characteristics of interest for study but lack complete genetic information.

To overcome these drawbacks, there have been attempts to generate sgRNA libraries through sequential enzymatic reactions using the genetic material of the organisms under study. Since this method does not rely solely on the genome sequence information itself, both the time and cost are greatly reduced, and further the enzymatic reactions used in the generation process are independent of the genetic material sequence of the target organism, making it theoretically applicable to all organisms.

However, these prior attempts had several significant shortcomings that made them unsuitable for phenotypic screening of prokaryotes, including bacteria. First, existing methods cannot generate guide RNA that targets only the non-template strand of a target gene. As described above, when conducting phenotypic-based screening using CRISPR for bacteria, it is essential to utilize the CRISPRi system.

Here, when dCas9 targets the coding sequence (CDS) of a gene, the sgRNA must complementarily bind to the non-template strand to block the progression of RNA polymerase, thus effectively achieving gene suppression. As described above, since enzyme-based library generation methods are not design-driven, it is inevitable that most sgRNAs are probabilistically engineered to target the CDS, and therefore it is very critical to have strand selectivity.

Second, most enzymatic reaction-based sgRNA production methods are not able to utilize RNA as genetic material. RNA is valuable as a starting material for sgRNA library construction because it shows the adjusted gene ratio according to the transcription level of each gene, but it has been rarely utilized due to the difficulty of isolating and purifying only mRNA containing various gene information from rRNA, which accounts for 85% or more of the total RNA.

In particular, unlike eukaryotic mRNA, prokaryotic mRNA lack structural features such as a poly A tail, making isolation and purification more difficult, and thus has not been used as a starting material.

Third, there were many types of restriction enzymes using conventional methods, and the reaction process was too long to be utilized in practice.

Therefore, it is necessary to develop a method for generating an sgRNA library capable of targeting only the non-template strand, utilizing mRNA as a starting material, being applied to all organisms including prokaryotes, and having a simplified enzymatic reaction process in order to solve the shortcomings of the above-described methods for generating an enzymatic reaction-based sgRNA library.

**[Disclosure]**

[Technical Problem]

An object of the present disclosure is to provide a primer set for generating an sgRNA library.

Another object of the present disclosure is to provide a method for generating an sgRNA library.

Still another object of the present disclosure is to provide uses of the sgRNA library produced by the method described above.

[Technical Solution]

In the following specification, description of overlapping content will be omitted to prevent any potential confusion arising from redundancy. In other words, the content of the invention is not limited to the following content; rather, it should be construed in accordance with the comprehensive content of the invention.

Further, terms used herein are merely used for illustration purposes, which should not be construed as limiting the present disclosure. Singular expressions include plural expressions unless the context clearly indicates otherwise. In the present specification, terms such as "comprise" or "have" are intended to designate the presence of features, numbers, steps, operations, components, parts, or combinations thereof described in the specification and it should not be understood as precluding the possibility of the presence or addition of one or more other features, numbers, steps, operations, components, parts, or combinations thereof.

Further, unless defined otherwise, all terms used herein, including technical or scientific terms, have the same meaning as commonly understood by one of ordinary skill in the art to which the embodiments belong. Terms such as those defined in commonly used dictionaries should be interpreted as having a meaning consistent with the meaning in the context of the related art, and unless explicitly defined in the present application, it should not to be construed in an idealized or overly formal sense.

In one general aspect, there is provided a primer set for generating an sgRNA library, comprising: a 1st adapter-binding primer that acts as a reverse transcription primer using fragmented mRNA of a target organism as a template and binds a 1st adapter to the 5' end of cDNA; and a 2nd adapter-binding primer that acts as a template strand extension primer of a reverse transcription product and binds a 2nd adapter to the 3' end of cDNA, wherein

(1) the 1st adapter-binding primer has a structure of the following General Formula I-1;

$$5'-X-Y-Z-3' \qquad (I\text{-}1)$$

wherein X is a site that functions as a primer for PCR I amplification, comprising 9 to 14 bases; Y is a type III restriction enzyme recognition site, comprising 6 bases; and Z is a random heptamer site having a hybridization sequence complementary to the fragmented mRNA of the target organism, comprising 7 bases; and
(2) the 2nd adapter-binding primer is a DNA-RNA chimeric primer, and has a structure of the following General Formula I-2;

$$5'-X'-Y'-Z'-3' \qquad (I\text{-}2)$$

wherein X' is a binding site of the primer in PCR I amplification, comprising 16 to 20 bases; Y' is a partial recognition site of any one of restriction enzymes SfiI, BamHI, or KpnI, comprising 8 bases; and Z' is a site that extends a strand (template) through switching mechanism at 5' end of RNA template (SMART), as an RNA sequence that hybridizes complementarily with an overhang at 3' end of the reverse transcription product, comprising three guanine (G) bases;
wherein the 2nd adapter-binding primer employs as a template, the reverse transcription product, which is obtained after performing reverse transcription using the 1st adapter-binding primer;
the 1st adapter acts as a type III restriction enzyme recognition site; and
the 2nd adapter acts as a partial recognition site of any one of restriction enzymes SfiI, BamHI, and KpnI.

Here, since X of the General Formula I-1, together with Y, serves to match the typical primer length of about ~20 mer and Tm, any combination of sequences for performing the role of a primer for Tm may be used irrespectively.
Here, the recognition site of any one restriction enzyme selected from SfiI, BamHI and KpnI may be appropriately selected for selection of the PAM sequence on mRNA. In other words, during the library generation process according to the present disclosure, the recognition site of restriction enzyme for PAM sequence selection is appropriately selected as the recognition site of any one restriction enzyme selected from SfiI, BamHI, and KpnI, and the 2nd adapter sequence may be changed to match the partial recognition site of the specific restriction enzyme selected in this manner, or the 2nd adapter having a universal sequence may be produced so as to include partial recognition sites of all of the above restriction enzymes. This allows for the generation of an appropriate sgRNA library tailored to the type of Cas9 used. In other words, the design may generate a specific or universal sgRNA library that is not limited to just one type of Cas9.
For example, the description of 'any one of restriction enzymes SfiI, BamHI, or KpnI' below means that after an enzymatic reaction is performed using a specific restriction enzyme (for example, any one selected from SfiI, BamHI, and KpnI) that recognizes a recognition site of the restriction enzyme, an sgRNA library for a specific Cas9 is generated, wherein the combination of the restriction enzyme for generating each sgRNA library and the Cas9 to be applied to the sgRNA library may be provided as follows: SpCas9- SfiI; SaCas9- BamHI; CjCas9- KpnI.

In the present disclosure, the 1st adapter-binding primer may further include a spacer sequence comprising 1 to 6 bases between Y and Z in the structure of General Formula I-1, if necessary. Here, the spacer sequence may be included to provide space between the type III restriction enzyme recognition site (Y) and the random heptamer site (Z) having a hybridization sequence complementary to the fragmented mRNA of the target organism.

In the present disclosure, the 1st adapter-binding DNA primer acts as a reverse transcription primer by complementarily binding to the mRNA fragment of the target organism and includes a type III restriction enzyme recognition site. Here, the type III restriction enzyme recognition site may preferably be an EcoP15I restriction enzyme recognition site. This EcoP15I restriction enzyme recognition site may have the sequence of **CAGCAG.** According to an embodiment of the present disclosure, SEQ ID NO: 1 (5'-AGGTATCTCGAGTCCC**CAGCAG**CNNNNNNN-3') including the restriction enzyme recognition site of CAGCAG was used.

In the present disclosure, the DNA-RNA chimeric primer for binding the 2nd adapter acts as a secondary template during reverse transcription and includes a partial recognition site of any one restriction enzyme selected from restriction enzymes SfiI, BamHI, and KpnI. According to an embodiment of the present disclosure, as an example, SEQ ID NO: 2 (5'-AGTGGTATCAACGCAGAGT*GGCC*AAGCG<u>*GG*</u> - 3'; SfiI), SEQ ID NO: 3 (AGTGGTATCAACGCAGAGTGGC-CAAGCG<u>*GG*</u>; BamHI), and SEQ ID NO: 4 (AGTGGTATCAACGCAGAGTGGCCAAGCG<u>*G*</u><u>G</u>; KpnI) may be included (bold italics indicate partial recognition sites of each restriction enzyme, and underlined <u>G</u> indicates RNA).

In a further description of the reverse transcription process by the 1st and 2nd adapters, the 1st adapter-binding primer acts as the reverse transcription primer by complementarily binding to the mRNA fragment via the random heptamer sequence at the 3' end, thereby initiating reverse transcription. Here, as an example, when using a reverse transcriptase of the M-MLV (Moloney murine leukemia virus) series, CCC is generated as an overhang at the 3' end of the cDNA when reverse transcription progresses to the end.

Here, when the RNA sequence present at the 3' end of the 2nd adapter complementarily binds to the overhang at the 3' end of the cDNA, the M-MLV series reverse transcriptase continues reverse transcription using the cDNA ligated to the 2nd adapter as a new template.

As a result, cDNA is generated from the mRNA fragment, while the 1st and 2nd adapters are attached to both ends of the mRNA-derived sequence, enabling subsequent PCR amplification and enzymatic reaction.

In another general aspect, there is provided a primer set for generating an sgRNA library, comprising: a 3rd adapter-generating forward primer (A3F) and a 3rd adapter-generating reverse primer (A3R) that are hybridized with a restriction enzyme cleavage product generated using the 1st adapter- and 2nd adapter-binding primer set, followed by ligation, wherein

(1) the 3rd adapter-generating forward primer (A3F) has a structure of the following General Formula II-1;

$$\text{5'-biotin-X''-Y''-Z''-1-3'} \qquad \text{(II-1)}$$

wherein X" is a binding site of the primer in PCR II amplification; Y" is a type IIS restriction enzyme recognition site, comprising 6 bases; Z" is a type III restriction enzyme recognition site, comprising 6 bases; and I is a site having a hybridization sequence complementary to the restriction enzyme cleavage product, comprising 1 to 4 bases; and
(2) the 3rd adapter-generating reverse primer (A3R) has a structure of the following General Formula II-2;

$$\text{5'-Z'''-Y'''-X'''-3'} \qquad \text{(II-2)}$$

wherein Z''' is a site where a type III restriction enzyme recognition site is generated by binding to the restriction enzyme cleavage product; Y''' is a type IIS restriction enzyme recognition site, comprising 6 bases; and X''' is a binding site of the primer in PCR II amplification;
the 3rd adapter is a double-stranded DNA to which biotin is attached, having the structure of the following General Formula II-3;

$$\text{5'-biotin-X*-Y*-Z*-3'} \qquad \text{(II-3)}$$

wherein X* is a type IIS restriction enzyme recognition site for cleavage between the 3rd adapter and the restriction enzyme cleavage product generated using the 1st adapter and 2nd adapter-binding primer set; Y* is a type III restriction enzyme recognition site; and Z* is a site for ligation with the restriction enzyme cleavage product generated using the 1st adapter and 2nd adapter-binding primer set; and

the 3rd adapter acts as a label site for separating a protospacer adjacent motif (PAM) adjacent sequence.

In the present disclosure, the 3rd adapter-generating forward primer (A3F) is selectively ligated to any one cleavage product selected from SfiI, BamHI and KpnI, includes biotin at the 5'-end, includes a type IIS restriction enzyme recognition site and a type III restriction enzyme recognition site, and includes a hybridization sequence complementary to any one cleavage product selected from SfiI, BamHI and KpnI at the 3'-end. Here, the type IIS restriction enzyme recognition site and the type III restriction enzyme recognition site may preferably be AcuI (restriction enzyme recognition site: 5'-CTGAAG-3') and EcoP15I (restriction enzyme recognition site: 5'-CAGCAG-3') restriction enzyme recognition sites, respectively.

For example, the 3rd adapter-generating forward primer may comprise any one selected from SEQ ID NO: 5 (5'-Biotin-AGGCTCACTGCATATG<u>CTGAAG</u>T**CAGCAG**C-3': a primer sequence ligated to a SfiI cleavage product), SEQ ID NO: 6 (5'-Biotin- AGGCTCACTGCATA<u>CTGAAG</u>TGT**CAGCA**-3': a primer sequence ligated to a BamHI cleavage product), and SEQ ID NO: 7 (5'-Biotin-AGGCTCACTGCATATG<u>CTGAAG</u>T**CAGCAG**GTAC-3': a primer sequence ligated to a KpnI cleavage product) (the underlined sequence indicates the AcuI recognition site, and the bold sequence indicates the EcoP15I recognition site).

Here, the 3rd adapter-generating forward primer of SEQ ID NO: 6, unlike SEQ ID NOs: 5 and 7, contains only a part of the type III restriction enzyme recognition site. This is because the SEQ ID NO: 6 is ligated in front of the G at the 5' end of the BamH I cleavage product generated by cleavage by BamH I, and the BamH I cleavage product to which the 3rd adapter-generating forward primer is ligated has a sequence of, for example, 5'-Biotin-AGGCTCACTGCATA<u>CTGAAG</u>TGT**CAGCAG**~~~-3', thereby finally completing the type III restriction enzyme recognition site. This particular sequence placement of SEQ ID NO: 6 is intended to match the optimal spacer length for SaCas9.

In the present disclosure, the 3rd adapter-generating reverse primer (A3R) is a sequence that hybridizes complementarily with the 3rd adapter-generating forward primer (A3F) that is selectively ligated to any one cleavage product selected from SfiI, BamHI, and KpnI, that is, a sequence that hybridizes with the remaining sequence except for a portion having a hybridization sequence complementary to the restriction enzyme cleavage product of the 3rd adapter-generating forward primer (A3F), and does not contain biotin. For example, the 3rd adapter-generating reverse primer (A3R) may comprise any one selected from SEQ ID NO: 8 (5'-GCTGACTTCAGCATATGCAGTGAGCCT-3'), SEQ ID NO: 9 (5'-GATCTGCTGACACTTCAGTATGCAGTGAGCCT-3'), and SEQ ID NO: 10 (5'-TGCTGACTTCAGCATATGCAGTGAGCCT-3').

Here, SEQ ID NOs: 5 and 8, SEQ ID NOs: 6 and 9, and SEQ ID NOs: 7 and 10 are applied as one set. The above SEQ ID NOs: 5 and 8 are applied when producing an sgRNA library for applying SpCas9 (*Streptococcus pyogenes* Cas9), the above SEQ ID NOs: 6 and 9 are applied when producing an sgRNA library for applying SaCas9 (*Staphylococcus aureus* Cas9), and the above SEQ ID NOs: 7 and 10 are applied when producing an sgRNA library for applying CjCas9 (*Campylobacter jejuni* Cas9).

The term "ligation" is a general term and is to be understood to include any method of linking a DNA product and a primer by a covalent bond.

The ligation reaction of the present disclosure may be performed using a wide variety of ligation agents, including enzymatic ligation agents and non-enzymatic ligation agents (e.g., chemical agents and photoligation agents), but according to a preferred embodiment of the present disclosure, the ligation reaction may be by an enzymatic ligation agent.

Here, the enzymatic ligation is performed by one ligation agent selected from the group consisting of SplintR ligase, bacteriophage T4 ligase, *E. coli* ligase, Afu ligase, Taq ligase, Tfl ligase, Mth ligase, Tth ligase, Tth HB8 ligase, Thermus species AK16D ligase, Ape ligase, LigTk ligase, Aae ligase, Rm ligase, Pfu ligase, ribozyme, and variants thereof.

The DNA product ligated with the 3rd adapter, generated using the 3rd adapter-generating primer, binds to magnetic beads into which streptavidin is introduced through the 5' end biotin, and when the beads are separated from the supernatant using a device such as a magnetic stand, only the PAM adjacent sequence may be separated as a result.

In another general aspect, there is provided a primer set for generating an sgRNA library, comprising: a 4th adapter-generating forward primer (A4F); a 4th adapter-generating reverse primer (A4R); a 5th adapter-generating forward primer (A5F); and a 5th adapter-generating reverse primer (A5R), which are ligated to both ends of the DNA produced by sequentially cleaving the double-stranded DNA product to which the 3rd adapter is ligated using the 3rd adapter-generating primer set, wherein

(1) the 4th adapter-generating forward primer (A4F) comprises a promoter sequence for transcription of an sgRNA spacer and 15 to 40 bases for performing Gibson assembly with an sgRNA-expressing vector;

(2) the 4th adapter-generating reverse primer (A4R) has a structure of the following III-1;

5'-A-B-3'    (III-1)

wherein A is a site having a complementary hybridization sequence comprising 2 bases with an overhang at the 5' end of a single-stranded DNA comprising 20 bases; and B is a site having a hybridization sequence complementary to the 4th adapter-generating forward primer (A4F) ;

(3) the 5th adapter-generating forward primer (A5F) comprises an sgRNA scaffold sequence and 15 to 40 bases for performing Gibson assembly with the sgRNA-expressing vector;

(4) the 5th adapter-generating reverse primer (A5R) has a structure of the following III-2;

$$5'-A'-B'-3' \qquad (III-2)$$

wherein A' is a site having a hybridization sequence complementary to the 5th adapter-generating forward primer (A5F); and B' is a site having a complementary hybridization sequence comprising 2 bases with an overhang of the 3' end of a single-stranded DNA comprising 20 bases;

the 4th adapter acts as a promoter site for sgRNA expression; and

the 5th adapter has an sgRNA scaffold sequence.

The 4th and 5th adapters serve as regions for Gibson assembly-based cloning into plasmids.

Specifically, the 'DNA produced by sequentially cleaving the double-stranded DNA product to which the 3rd adapter is ligated using the 3rd adapter-generating primer set' is 'a 20 bp double-stranded DNA product with 2 nt overhangs on both sides by complementary binding of 16 nt and 20 nt DNA', i.e., 'a 20 bp sgRNA spacer'.

At this time, the 16nt sequence is the same sequence as the mRNA sequence (the same sequence as the non-template strand of the gene), and the 20nt sequence is its complementary sequence. By ligating the 20nt sequence between the promoter sequence of the 4th adapter and the sgRNA scaffold sequence of the 5th adapter, using the 2nt overhangs that present only at both ends of the 20nt sequence, the 20nt sequence is transcribed as an sgRNA spacer by the promoter, which binds to the non-template strand of the target gene.

In one example, the 4th adapter-generating forward primer (A4F) may comprise SEQ ID NO: 11, and the 4th adapter-generating reverse primer (A4R) may comprise SEQ ID NO: 12.

In one example, the 5th adapter-generating forward primer (A5F) may comprise SEQ ID NO: 13, and the 5th adapter-generating reverse primer (A5R) may comprise SEQ ID NO: 14.

In another general aspect, there is provided a method for generating an sgRNA library comprising: (a) fragmenting mRNA of a target organism; (b) synthesizing cDNA, using the fragmented mRNA as a template, comprising a 1st adapter that acts as a reverse transcription PCR primer binding site at the 5' end and a 2nd adapter that acts as a recognition site of any one restriction enzyme selected from SfiI, BamHI, and KpnI at the 3' end; (c) obtaining a restriction enzyme treatment product by treatment with any one restriction enzyme selected from SfiI, BamHI, and KpnI; (d) ligating a 3rd adapter that forms a complementary hybridization with an overhang at the 3' end of the restriction enzyme treatment product of Step (c) and acts as a labeling site for separating a protospacer adjacent motif (PAM) adjacent sequence; (e) selecting only cDNA to which the 3rd adapter is ligated; (f) treating with a type III restriction enzyme; and (g) producing sgRNA spacer DNA by treatment with type II S restriction enzyme.

Here, the fragmenting of the mRNA in Step (a) may be performed to expose the promoter adjacent motif (PAM) complementary sequence at the 5' end of the mRNA. The PAM sequence acts as a kind of notice board in the CRISPRi system to help Cas9 recognize the target DNA for cleavage.

Further, the fragmenting of the mRNA is performed to convert the mRNA, which originally has a variable length distribution, into fragments of relatively constant length, preferably around 100 nt, thereby facilitating the design and validation of subsequent enzymatic reactions.

The fragmenting of the mRNA in Step (a) is performed by including the following steps: i) extracting total RNA of the target organism; ii) generating an rRNA-DNA double strand by treatment with a DNA probe having a sequence complementary to rRNA; and iii) removing rRNA by treatment with an RNase that selectively degrades only rRNA of the rRNA-DNA double strand of Step ii).

Here, the RNase is a thermostable RNaseH.

In the present disclosure, the fragmenting of the mRNA may be performed by any one selected from the group consisting of physical fragmentation, enzyme-based fragmentation, and chemical fragmentation.

The mechanical fragmentation may be performed through acoustic shearing and sonication, which are methods of fragmenting DNA or RNA by focusing short-wavelength, highfrequency acoustic energy on a sample. The cleavage may be performed between 150 bp and several kb, where the reaction is easy and fast, but the use of specialized equipment is required.

The enzyme-based fragmentation is low in equipment dependence, but may cause a cleavage site bias issue due to restriction enzyme recognition sites or sequence preference issues. In the case of DNA, various restriction enzyme recognition sites or non-specific nucleases (DNase I), etc., are present, which may be used for enzyme-based fragmentation. In the case of RNA, however, RNase III may be used, but since this only employs dsRNA as a substrate, it cannot be used for mRNA fragmentation, making practical use difficult when targeting RNA.

Chemical fragmentation is performed by reacting DNA or RNA with metal cations such as $Mg^{2+}$ and $Zn^{2+}$ to cleave a phosphodiester bond. The chemical fragmentation has the advantage of controllable fragment sizes between a few hundred nt by controlling the reaction time, being less instrument dependent, and being less prone to the cleavage site bias issue.

In the present disclosure, mRNA fragmentation may be performed more preferably by using chemical fragmentation.

More specifically, in an embodiment of the present disclosure, a reduction in reaction time compared to mRNA fragmentation using a restriction enzyme was achieved by using a method of heating with $Mg^{2+}$ at a high temperature.

Here, Step (b) may be performed to conduct reverse transcription of the fragmented mRNA and synthesize cDNA containing a recognition site of any one restriction enzyme selected from SfiI, BamHI, and KpnI.

Here, the treatment with any one restriction enzyme selected from SfiI, BamHI, and KpnI as in Step (c) above enables selective cleavage of only the PAM adjacent sequence. In other words, in the case of cDNA having a PAM complementary sequence at the 5' end of the mRNA used as a template in the reverse transcription process of Step (b) above, the partial restriction enzyme recognition site present in the 2nd adapter is followed by the PAM complementary sequence to form a complete restriction enzyme recognition site as shown in General Formulas I-3, I-4, and I-5 below.

5'-<u>X'</u>-G**GGCC**N-(omitted)-3' (I-3; when producing sgRNA library for SpCas9 application)
5'-<u>X'</u>-G**GGAYYC**N-(omitted)-3' (I-4; when producing sgRNA library for SaCas9 application)
5'-<u>X'</u>-GG**GGTRYN**NNN-(omitted)-3' (I-5; when producing sgRNA library for cjCas9 application)

Therefore, only the PAM adjacent sequences that satisfy the structures as shown in General Formulas I-3 to I-5 above, are cleaved by the restriction enzyme.

Here, Step (d) above may be performed to bind biotin to cDNA containing the PAM adjacent sequence, and Step (e) above may be performed to specifically screen only cDNA containing the PAM adjacent sequence. Here, the selecting of cDNA is performed using biotin-streptavidin binding. In other words, the biotin-streptavidin binding in which the biotin-bound cDNA containing a PAM adjacent sequence is bound to the streptavidin magnetic beads, may achieve specific screening and enrichment of only cDNA containing PAM adjacent sequence.

Here, sequential enzymatic reactions through Steps (f) and (g) may be performed to generate the sequence corresponding to the spacer region of sgRNA.

The cDNA product obtained from Step (d) above contains the 1st adapter at the 5' end and the 3rd adapter at the 3' end, and as the type III restriction enzyme recognizes the type III restriction enzyme recognition site present in the 1st and 3rd adapters, an mRNA-derived sequence, located at a certain distance from the adapter, is cleaved.

Subsequently, as the type II S restriction enzyme recognizes the type II S restriction enzyme recognition site present on the 3rd adapter, the portion between the 3rd adapter and the mRNA-derived sequence is cleaved, and finally, the 16-18 nt DNA complementarily binds to the 20-22 nt DNA to generate a 20-22 bp double-stranded DNA product with 2 nt overhangs on both sides. Through the above process, a 20-22 bp sgRNA spacer sequence randomly derived from the genome sequence is generated. Specifically, when SpCas9 (*Streptococcus pyogenes* Cas9) or CjCas9 (*Campylobacter jejuni* Cas9) is used to produce an sgRNA library, 16nt and 20nt DNAs complementarily bind to generate the 20 bp double-stranded DNA product with 2nt overhangs on both sides, and when SaCas9 (*Staphylococcus aureus* Cas9) is used to produce an sgRNA library, 18nt and 22nt DNAs complementarily bind to generate a 22 bp double-stranded DNA product with 2nt overhangs on both sides.

Here, the 16nt (18nt) sequence is the same sequence as the mRNA sequence (the same sequence as the non-template strand of the gene), and the 20nt (22nt) sequence is its complementary sequence.

In other words, the method for generating the sgRNA library according to the present disclosure may produce DNA sequences corresponding to the optimal spacer length of cas9, which is 20 bp (SpCas9 or CjCas9) to 22 bp (SaCas9), while targeting the PAM adjacent region with only three restriction enzymatic reactions. In this case, the sgRNA spacer DNA is produced by the complementary binding of a single-stranded DNA comprising 20 (SpCas9 or CjCas9) or 22 (SaCas9) bases and a single-stranded DNA comprising 16 (SpCas9 or CjCas9) or 18 (SaCas9) bases.

Meanwhile, in the method for generating an sgRNA library according to the present disclosure, after the process of binding each adapter, amplifying the corresponding product through PCR is further performed. In other words, PCR I may be performed between Steps (b) and (c) to obtain and amplify cDNA to which the 1st adapter and the 2nd adapter are ligated, and PCR II may be performed between Steps (e) and (f) to amplify cDNA to which the 3rd adapter is ligated.

Meanwhile, the method for generating an sgRNA library according to the present disclosure may further comprise: (h) ligating the 4th adapter that acts as a promoter for transcription and the 5th adapter that acts as an sgRNA scaffold, to both ends of the sgRNA spacer DNA; and (i) producing an sgRNA library in a plasmid state, comprising DNA excluding the sgRNA spacer sequence as a vector and DNA containing the sgRNA spacer sequence as an insert.

The above process is performed on a 20-base single-stranded DNA in sgRNA spacer DNA. This process may allow the sgRNA to bind to the non-template strand by controlling the sequence transcribed by the actual promoter through setting the overhang orientation of the adapter during the process of ligating the sequence with the Gibson assembly adapter.

Specifically, when ligation is performed so that the 20-base single-stranded DNA is placed between the promoter sequence of the 4th adapter and the sgRNA scaffold sequence of the 5th adapter by using the overhang containing two bases that exist only at both ends of the 20-base single-stranded DNA, the 20-base single-stranded DNA is transcribed as an sgRNA spacer by the promoter, which binds to the non-template strand of the target gene.

In other words, it is possible to generate an sgRNA library for CRISPRi that targets only the non-template strand by controlling the ligation orientation.

Then, Gibson assembly is performed on the plasmid for transcribing sgRNA, thereby completing the sgRNA library in a plasmid state.

Accordingly, the sgRNA library according to the present disclosure may target the entire genome of target organism without relying solely on genome sequence information by targeting only the non-template strand.

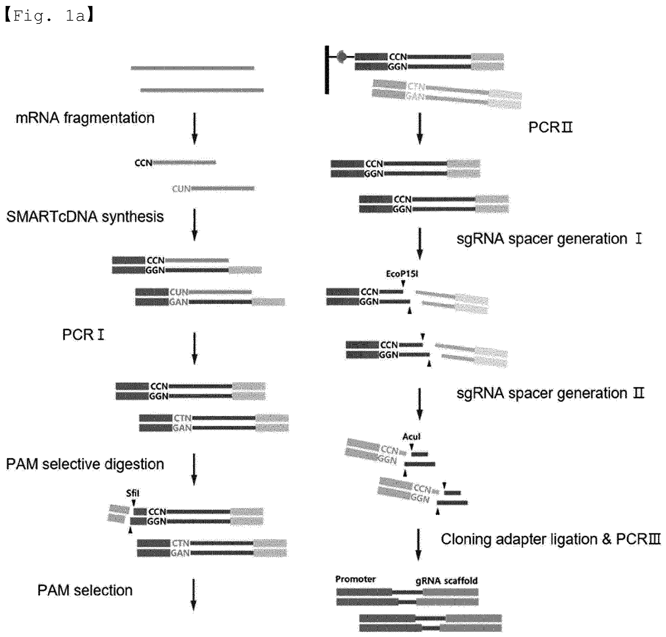

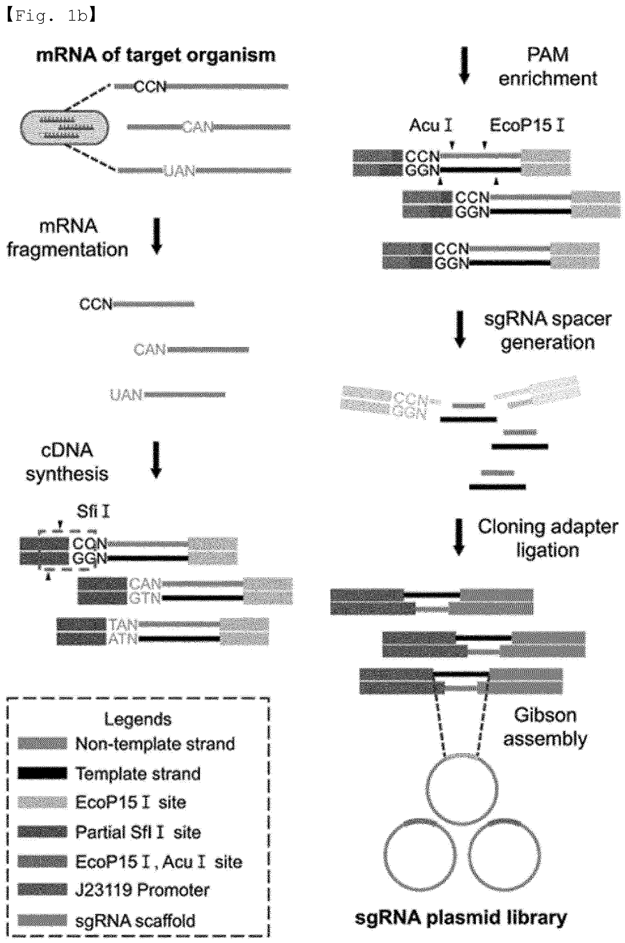

The overall process of generating an sgRNA library according to the present disclosure is shown in FIG. 1 (a and b).

To be more specific about this, the method for generating an sgRNA library according to the present disclosure may comprise steps of 1) mRNA fragmentation, 2) SMART cDNA synthesis (binding of 1st and 2nd adapters), 3) PCR I, 4) cleavage of a SfiI (or BamHI or KpnI) restriction enzyme recognition site, 5) 3rd adapter ligation and selection and enrichment of DNA containing a PAM complementary sequence, 6) PCR II, 7) generation of a restriction enzyme-based sgRNA spacer sequence, and 8) cloning adapter ligation, followed by PCR III and Gibson assembly.

1) The mRNA fragmentation proceeds to a length of about 100 nt, and exposes the PAM complementary sequence at the 5' end of the cDNA synthesized below, allowing for screening enrichment while simultaneously facilitating subsequent library production.

2) The SMART cDNA synthesis is a process of synthesizing cDNA as a template strand, in which the 1st adapter that acts as a type III restriction enzyme recognition site and the 2nd adapter that acts as a SfiI (or BamHI or KpnI) restriction enzyme recognition site are ligated to the fragmented mRNA described above. Specifically, seven random sequences at the 3' end of the 1st adapter-binding primer may bind to the fragmented mRNA and act as the primer to perform reverse transcription by reverse transcriptase, and its activity may cause the addition of a three cytosine (CCC) overhang to the 3' end of cDNA, and when three RNA guanines (GGG) at the 3' end of the 2nd adapter-binding primer bind to this overhang, reverse transcription may continue, resulting in generating cDNA having the 1st and 2nd adapters attached to both ends.

3) The PCR I is a process of amplifying the synthesized cDNA. Specifically, PCR may be performed using the synthesized cDNA as a template and the 1st and 2nd adapters as primer regions, thereby effectively amplifying the cDNA to which the 1st and 2nd adapters are ligated.

4) The cleavage of SfiI (or BamHI or KpnI) restriction enzyme recognition site is a process to selectively cleave only the PAM adjacent sequence at the 5' end of the synthesized cDNA. This is possible because in the case of cDNA, which has a PAM complementary sequence at the 5' end of the mRNA used as a template in the reverse transcription process, the partial restriction enzyme recognition site present in the 2nd adapter and the PAM complementary sequence are connected to form a complete restriction enzyme recognition site. As a result, the 2nd adapter is cleaved. Here, different restriction enzyme recognition sites are matched depending on the type of Cas9 used. For example, the partial restriction enzyme recognition site of the 2nd adapter may be designed appropriately so that the SfiI restriction enzyme recognition site is located when producing the sgRNA library for applying SpCas9, the BamHI recognition site is located when producing the sgRNA library for applying SaCas9, and the KpnI recognition site is located when producing the sgRNA library for applying CjCas9.

5) The 3rd adapter ligation and selection and enrichment of DNA containing a PAM complementary sequence is a process of binding biotin to cDNA, cleaved by the SfiI (or BamHI or KpnI) restriction enzyme, and selecting and enriching only DNA containing the PAM complementary sequence. Specifically, when the overhang of cDNA cleaved by the restriction enzyme is ligated to the 3rd adapter having a sequence complementary to the overhang and containing biotin attached thereto, by amplification using the 3rd adapter-generating primer set, the 3rd adapter is linked only to cDNA containing the PAM complementary sequence, thereby achieving selection and enrichment of only cDNA containing the PAM complementary sequence by the antibody-antigen reaction of biotin-streptavidin. As a result, the 3rd adapter may be linked to the position of the cleaved 2nd adapter above, ultimately generating cDNA having the 1st and 3rd adapters attached to both ends.

6) The PCR II is a process of amplifying cDNA having the 1st and 3rd adapters attached to both ends. Specifically, PCR may be performed using the synthesized cDNA as a template and the 1st and 3nd adapters as primer regions, thereby effectively amplifying the cDNA to which the 1st and 3nd adapters are ligated.

7) The generation of a restriction enzyme-based sgRNA spacer sequence is a process for generating sgRNA spacer DNA in which 20- or 22-base single-stranded DNA and 16- or 18-base single-stranded DNA form a complementary binding for generating an sgRNA library. In detail, the cDNA to which the 1st and 3rd adapters are ligated may have a type III restriction enzyme recognition site in the 1st adapter and the 3rd adapter, and the 3rd adapter may further include a type IIS restriction enzyme recognition site. These enzymes have recognition sites and cleavage sites that are far apart from each other, resulting in the cleavage of mRNA-derived random sequences located at a defined distance from the adapter sequence. Accordingly, when the cleavage of type III restriction enzyme recognition site is performed and then the cleavage of type IIS restriction enzyme recognition site is sequentially performed, 20- or 22-base single-stranded DNA and 16- or 18-base single-stranded DNA complementarily bind to produce a double-stranded sgRNA spacer DNA with 2nt overhangs on both sides. Here, the 20- or 22-base single-stranded DNA is utilized as the sgRNA spacer sequence.

8) Cloning adapter ligation, PCR III, and Gibson assembly are to obtain the sgRNA library in a plasmid state. Specifically, the 4th adapter acting as a promoter and the 5th adapter acting as an sgRNA scaffold are connected by amplification using the 4th adapter- and 5th adapter-generating primer sets, respectively. Here, the sequence transcribed by the promoter is the sgRNA spacer sequence (20- or 22-base single-stranded DNA), which is the same sequence as the complementary sequence of the mRNA and the template strand of the genomic DNA, and thus the transcribed sgRNA binds to the non-template strand. Thereafter, PCR III may be performed to amplify the sgRNA spacer sequence to which the 4th and 5th adapters are ligated, and PCR may be performed on a portion of the plasmid excluding the sgRNA spacer sequence for Gibson assembly of the corresponding amplified product, thereby finally obtaining an sgRNA library in a plasmid state.

The sgRNA library according to the present disclosure may be analyzed for its composition and characteristics through Sanger sequencing and next-generation sequencing, and may be used by transformation on cells for which phenotype-based screening is actually desired.

Specifically, the phenotype-based screening, including application of the sgRNA library according to the present disclosure to the CRISPRi system, may be performed by additional transformation with a plasmid for sgRNA transcription on cells transformed to express Cas9 via genome integration or plasmid transformation.

Further, simultaneous expression of sgRNA and Cas9 may be induced with a single transformation by introducing a gene for Cas9 expression into a plasmid for sgRNA transcription.

Here, Cas9 for performing the CRISPRi system may be any one selected from the group consisting of SpCas9 (*Streptococcus pyogenes* Cas9; recognition site: 5'-GGCCNNNN/NGGCC-3'), SaCas9 (*Staphylococcus aureus* Cas9; recognition site: 5'-G/GATCC-3'), and CjCas9 (*Campylobacter jejuni* Cas9; recognition site: 5'-GGTAC/C-3').

Accordingly, the present disclosure provides a kit for phenotype-based screening comprising the sgRNA library produced by the method described herein.

The kit of the present disclosure may additionally include various polynucleotide molecules, enzymes, various buffers and reagents. The optimal amount of reagent to be used in any one particular reaction may be easily determined by those skilled in the art having learned the disclosure herein.

Typically, the kit of the present disclosure is manufactured in a separate package or compartment including the above described components.

The kit of the present disclosure is manufactured to perform the phenotype-based screening using the sgRNA library of the present disclosure described above, and thus redundant descriptions are omitted to avoid complexity of the present specification.

The present disclosure also provides a phenotype-based screening method using the sgRNA library produced by the method described above.

The phenotype-based screening method according to the present disclosure may be performed by transformation on cells of an actual target organism with the sgRNA library produced by the method described above.

The sgRNA library according to the present disclosure may be applied to all living organisms, including prokaryotes, and thus there is no limitation on the target organisms to which the sgRNA library according to the present disclosure is applied.

In addition, the sgRNA library according to the present disclosure may target the entire genome without relying solely on the genome sequence information of the target organism by targeting only the non-template strand, may target genes that are actually actively expressed by utilizing mRNA as a starting material, and may effectively resolve the shortcomings of the phenotype-based screening method performed using the conventional enzymatic reaction-based sgRNA library, through a simplified enzymatic reaction process.

[Advantageous Effects]

The method for generating a total mRNA-based random sgRNA library through an enzymatic reaction according to the present disclosure may generate an sgRNA library that may target the entire genome of any organism, including prokaryotes, without relying on genome sequence information and may be used for phenotype-based screening in prokaryotes because the generated sgRNAs selectively bind only to non-template strands of a target gene.

In addition, mRNA, which is a useful genetic material, as well as DNA may be used as a starting material, enabling the generation of an sgRNA library targeting an actively expressed gene.

[Description of Drawings]

FIGS. 1a and 1b are schematic diagrams showing the entire process of generating an sgRNA library by performing sequential enzymatic reactions based on an mRNA sample with rRNA removed from total RNA.

FIG. 2 shows the length distribution analysis of total RNA (total RNA) and samples with rRNA removed from total RNA extracted from the target organism from which sgRNA library is to be generated, analyzed by microfluidic electrophoresis (A: total RNA, B: samples with rRNA removed from total RNA).

FIG. 3 shows the length distribution analysis of an mRNA sample reacted with $Mg^{2+}$ at high temperature for a short time to generate fragments of about 100 nt, followed by microfluidic electrophoresis.

FIG. 4 shows the results of reverse transcription and PCR I, followed by electrophoresis based on the mRNA fragments.

FIG. 5 shows the SfiI reaction, 3rd adapter attachment, and magnetic bead-based separation of the PCR I product to select only PAM-adjacent sequences, followed by PCR II and electrophoresis.

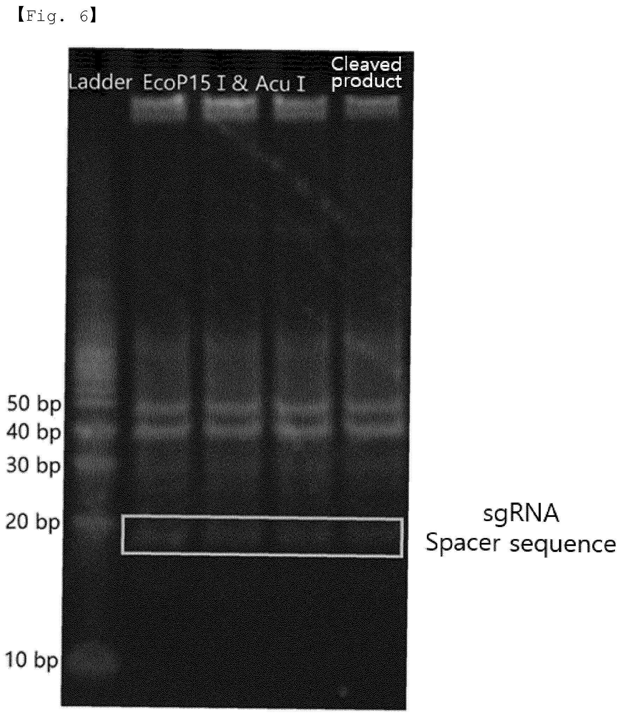

FIG. 6 shows the electrophoresis results of the PCR II product after a 20 bp sgRNA spacer sequence was generated through sequential restriction enzymatic reaction using EcoP15I and AcuI.

FIG. 7 shows the electrophoresis results of the sgRNA spacer sequence generated through the sequential restriction enzymatic reaction, ligated with 4th and 5th adapters for cloning, and amplified via PCR.

FIG. 8 shows the results of the analysis of the library by Sanger sequencing (A to D) and next-generation sequencing (E) (A: length distribution of the spacer portion of the product after Gibson assembly of the sgRNA sequence ligated with the cloning adapter onto a plasmid and transformed into *E. coli* K-12 MG1655, B: distribution of sgRNA target sequence types, C: PAM adjacency of sgRNA target sites, D: whether sgRNA non-template strand targeting occurred, and E: ratios of spacer length, RNA type, PAM adjacency, and strand selectivity).

FIG. 9 shows the results of the insertion and reference genome alignment ratio, genome coverage, and number of different sgRNAs after next-generation sequencing of the sgRNA library in a plasmid state.

FIG. 10 is a schematic diagram illustrating the process of screening essential genes by applying the sgRNA library generated by the method according to the present disclosure to phenotype-based screening and then observing changes in the ratio of sgRNA reads assigned to each gene by next-generation sequencing.

FIG. 11 shows the identification of sgRNA read count ratio changes per gene for all MG1655 genes and listed in order of $-\log_2$(fold change) [A: sgRNA read count ratio changes (the larger the $-\log_2$(fold change), the more growth stagnation occurs upon expression inhibition by dCas9), and B: listed in order of $-\log_2$(fold change)].

FIG. 12 shows a list of target genes whose ratio in dCas9-expressing cells was reduced by more than 4-fold ($-\log_2$(fold change) >2) compared to that in dCas9-unexpressing cells, and a comparison of this list with the list of essential genes in the Keio collection.

[Best Mode]

Hereinafter, the present disclosure will be described in more detail through Examples. These Examples are provided solely for the purpose of illustrating the present disclosure, and it will be apparent to those skilled in the art that the scope of the present disclosure should not be construed as limited by these Examples.

**Experimental Materials**

The kit for extracting total RNA used in the present experiment was purchased from New England Biolabs (NEB, Ipswich, MA, USA). Thermostable RNaseH used in the rRNA removal process was purchased from Lucigen (Middleton, WI, USA), and 5S, 16S, and 23S rRNA complementary DNA probes were synthesized and purchased from Integrated DNA Technologies, Inc. (IDT, Coralville, IW, USA). RNA purification kit and $Mg^{2+}$ fragmentation reagent were purchased from NEB (Ipswich, MA, USA). SuperScript™ II Reverse Transcriptase utilized for cDNA synthesis via the switching mechanism at the 5' end of the RNA transcript (SMART) was purchased from Thermo Fisher Scientific (Waltham, MA, USA), the 1st and 2nd adapters were synthesized from IDT (Coralville, IW, USA), and the betaine solution was purchased from Sigma-Aldrich (Burlington, MA, USA). RNase inhibitor murine was purchased from NEB (Ipswich, MA, USA). RNase H and RNaseI, used to remove RNA after SMART cDNA synthesis, were purchased from NEB (Ipswich, MA, USA). The purification kit used for purification of SMART cDNA synthesis products was purchased from Promega (Madison, WI, USA).

Q5 polymerase used in PCR I, PCR II, and PCR III was purchased from NEB (Ipswich, MA, USA), dNTPs were purchased from Takara (Kyoto, Japan), primers were synthesized by Cosmogenetech, Inc. (Seoul, Republic of Korea), and the kit used for purification of PCR products was purchased from GeneAll Biotechonology (Seoul, Republic of Korea). The sfiI restriction enzyme, used in the process of selectively cleaving sequences including PAM (Promoter adjacent motif), was purchased from NEB (Ipswich, MA, USA). The 3rd adapter, which was used for ligation of the products cleaved by SfiI, was synthesized by IDT (Coralville, IW, USA), and the quick ligase used for ligation was purchased from SolGent (Daejeon, Republic of Korea).

Streptavidin magnetic beads used to select only PAM-containing sequences by reacting with biotin attached to the 3rd adapter were purchased from Thermo Fisher Scientific (Waltham, MA, USA). EcoP15I and AcuI, which were used in the process of cleaving the PCR II product to the desired spacer length, were purchased from NEB (Ipswich, MA, USA) and Enzynomics (Daejeon, Republic of Korea), respectively. The PAGE recovery kit used to purify the cleavage products to the desired spacer length was purchased from Zymo research (Irvine, CA, USA). The 4th and 5th adapters ligated to the cleavage products were synthesized by IDT (Coralville, IW, USA), and the T4 ligase used in the ligation process was purchased from NEB (Ipswich, MA, USA).

The 2X Hifi DNA assembly master mix used for Gibson assembly of the 3rd PCR product was purchased from NEB (Ipswich, MA, USA). The purification kit used for purification of Gibson assembly products was purchased from GeneAll Biotechonology (Seoul, Republic of Korea). The kit for sgRNA plasmid extraction for Sanger sequencing was purchased from GeneAll Biotechonology (Seoul, Republic of Korea). A reagent kit for next-generation sequencing of sgRNA plasmids before and after phenotype-based screening was purchased from Illumina, Inc. (San Diego, CA, USA).

## Example 1. Total RNA extraction and mRNA selection and enrichment of target microorganisms for analysis

In the present Example, in order to secure starting materials for generating an sgRNA library, the target microorganism for analysis was cultured to extract total RNA, and a DNA probe having a sequence complementary to rRNA and heat-stable RNaseH were used to remove rRNA, thereby selecting and enriching only mRNA.

### 1-1. Extraction of total RNA from target microorganisms for analysis

As the target microorganism for analysis, *E. coli K*-12 MG1655, a representative bacterial model organism, was selected. First, cells were cultured in a shaking incubator at 37°C and 200 rpm using 100 ml of LB medium until the exponential growth phase (0.4-0.5 of optical density at 600 nm) was reached. After that, 20 ml of the culture medium was taken and divided into four tubes of 5 ml each, and the cells were collected by centrifugation, the supernatant was discarded, and the cells were resuspended using 100 $\mu$l of RNA extraction solution (18 mM EDTA, 0.025% SDS, 1% $\beta$-mercaptoethanol, and 95% formamide), and lysed by heating at 95°C for 7 minutes. Then, the reaction solution was centrifuged at 16,000 g for 5 minutes, then the supernatant was collected, and only RNA longer than 200 nt was selected and purified using a total RNA purification kit. The concentration of purified total RNA was measured using Qubit BR RNA.

### 1-2. mRNA selection and enrichment

In order to effectively conduct various experiments, including phenotype-based screening, using the sgRNA library presented in the present disclosure, the library must contain the entire genome information of the model organism, which is determined by the composition of the starting material for library production. The total RNA mass composition of prokaryotes, including bacteria, is dominated by rRNA (about 80%) and tRNA (about 17%), while mRNA, which contains the entire genome information, is only about 3%. Therefore, only mRNA should be selected and enriched from total RNA to produce high-quality sgRNA libraries.

Unlike eukaryotes, the mRNAs of prokaryotes such as bacteria lack structural features such as a poly A tail, which complicates selection and enrichment. This has historically limited the starting material for sgRNA library generation methods to DNA or restricted their applicability to eukaryotes.

In the present disclosure, 71 kinds and 37 kinds of DNA sequences complementary to 23S and 16S rRNA referenced in a previous study (Choe, D.; et. al. PLOS Genet. 2021, 17 (9), e1009821.) and 3 kinds of directly designed 5S rRNA complementary binding DNAs were utilized to form rRNA-DNA double-strand utilizing the sequences disclosed in Table 1 below, and then rRNA was digested using a thermostable RNaseH that selectively degrades only RNA forming DNA-RNA double-strand, followed by a purification method that selects only RNA of 200 nt or more, to remove rRNA fragments and tRNA, thereby selecting and concentrating only mRNA.

[Table 1]

| SEQ No. | Sequence Name | Sequence Information (5'→3') | Target |
|---------|---------------|------------------------------|--------|
| 15 | 16S_1 | CTTCTTCCTGTTACCGTTCGACTTGCATGTGT | 16S rRNA |
| 16 | 16S_1B | GCCTGCCGCCAGCGTTCAATCTGAGCCATGAT | |
| 17 | 16S_2 | CGTTTCCAGTAGTTATCCCCCTCCATCAGGCA | |
| 18 | 16S_2B | ACATTACTCACCCGTCCGCCACTCGTCAGCAA | |
| 19 | 16S_3 | TAATCCCATCTGGGCACATCCGATGGCAAGAG | |
| 20 | 16S_3B | TCCCCCTCTTTGGTCTTGCGACGTTATGCGGT | |
| 21 | 16S_4 | GTCTCAGTTCCAGTGTGGCTGGTCATCCTCTC | |
| 22 | 16S_4B | AGGGATCGTCGCCTAGGTGAGCCGTTACCCCA | |
| 23 | 16S_5 | CATACACGCGGCATGGCTGCATCAGGCTTGCG | |
| 24 | 16S_5B | CAATATTCCCCACTGCTGCCTCCCGTAGGAGT | |
| 25 | 16S_6 | GCGGGTAACGTCAATGAGCAAAGGTATTAACT | |
| 26 | 16S_6B | TCCTCCCCGCTGAAAGTACTTTACAACCCGAA | |
| 27 | 16S_7 | TTTACGCCCAGTAATTCCGATTAACGCTTGCA | |
| 28 | 16S 7B | TTACCGCGGCTGCTGGCACGGAGTTAGCCGGT | |
| 29 | 16S_8 | CTCAAGCTTGCCAGTATCAGATGCAGTTCCCA | |
| 30 | 16S_8B | CGGGGATTTCACATCTGACTTAACAAACCGCC | |

(continued)

| SEQ No. | Sequence Name | Sequence Information (5'→3') | Target |
|---------|---------------|------------------------------|--------|
| 31 | 16S_9 | GGGGCCGCCTTCGCCACCGGTATTCCTCCAGA | |
| 32 | 16S_9B | ATTTCACCGCTACACCTGGAATTCTACCCCCC | |
| 33 | 16S_10 | ATCGTTTACGGCGTGGACTACCAGGGTATCTA | |
| 34 | 16S_10B | GCTCCCCACGCTTTCGCACCTGAGCGTCAGTC | |
| 35 | 16S_11 | CTTGCGGCCGTACTCCCCAGGCGGTCGACTTA | |
| 36 | 16S_11B | CTCCGGAAGCCACGCCTCAAGGGCACAACCTC | |
| 37 | 16S_12 | GTAAGGTTCTTCGCGTTGCATCGAATTAAACC | |
| 38 | 16S 12B | ACCGCTTGTGCGGGCCCCCGTCAATTCATTTG | |
| 39 | 16S_13 | GACGACAGCCATGCAGCACCTGTCTCACGGTT | |
| 40 | 16S_13B | ACATTCTCATCTCTGAAAACTTCCGTGGATGT | |
| 41 | 16S_14 | AGTTCCCGGCCGGACCGCTGGCAACAAAGGAT | |
| 42 | 16S_14B | GCTCGTTGCGGGACTTAACCCAACATTTCACA | |
| 43 | 16S_15 | GTGTGTAGCCCTGGTCGTAAGGGCCATGATGA | |
| 44 | 16S_15B | ATCCCCACCTTCCTCCAGTTTATCACTGGCAG | |
| 45 | 16S_16 | CTCCAATCCGGACTACGACGCACTTTATGAGG | |
| 46 | 16S_16B | TCTCGCGAGGTCGCTTCTCTTTGTATGCGCCA | |
| 47 | 16S_17 | TACAAGGCCCGGGAACGTATTCACCGTGGCAT | |

(continued)

| SEQ No. | Sequence Name | Sequence Information (5'→3') | Target |
|---|---|---|---|
| 48 | 16S_17B | CGATTACTAGCGATTCCGACTTCATGGAGTCG | |
| 49 | 16S_18 | TCACAAAGTGGTAAGCGCCCTCCCGAAGGTTA | |
| 50 | 16S_18B | CTTCTTTTGCAACCCACTCCCATGGTGTGACG | |
| 51 | 16S_19 | CCGCAGGTTCCCCTACGGTTACCTTGTTACGA | |
| 52 | 23S_1 | ACCGACGCTTATCGCAGATTAGCACGTCCTTC | 23S rRNA |
| 53 | 23S_1B | GACTGCCAGGGCATCCACCGTGTACGCTTAGT | |
| 54 | 23S_2 | GGATTCAGTTAATGATAGTGTGTCGAAACACA | |
| 55 | 23S_2B | CCCATTCGGAAATCGCCGGTTATAACGGTTCA | |
| 56 | 23S_3 | GCTACTGGGGGAATCTCGGTTGATTTCTTTTC | |
| 57 | 23S_3B | CTTAGATGTTTCAGTTCCCCCGGTTCGCCTCA | |
| 58 | 23S_4 | ACCCTGTATCGCGCGCCTTTCCAGACGCTTCC | |
| 59 | 23S_4B | CACACTGATTCAGGCTCTGGGCTGCTCCCCGT | |
| 60 | 23S_5 | CCCCCATATTCAGACAGGATACCACGTGTCCC | |
| 61 | 23S_5B | ATCGAGCTCACAGCATGTGCATTTTTGTGTAC | |
| 62 | 23S_6 | TCGCCGGGGTTCTTTTCGCCTTTCCCTCACGG | |
| 63 | 23S_6B | ACTATCGGTCAGTCAGGAGTATTTAGCCTTGG | |
| 64 | 23S_7 | TATACAAAAGGTACGCAGTCACACGCCTAAGC | |

(continued)

| SEQ No. | Sequence Name | Sequence Information (5'→3') | Target |
|---------|---------------|------------------------------|--------|
| 65 | 23S_7B | CTGCTTGTACGTACACGGTTTCAGGTTCT TTT | |
| 66 | 23S_8 | CTTAACGCCCAGTTAAGACTCGGTTTCCC TTC | |
| 67 | 23S_8B | ATTCGGTTAACCTTGCTACAGAATATAAG TCG | |
| 68 | 23S_9 | CGGTTCGGTCCTCCAGTTAGTGTTACCCA ACC | |
| 69 | 23S_9B | CCCATGGCTAGATCACCGGGTTTCGGGTC TAT | |
| 70 | 23S_10 | TTCGGGGAGAACCAGCTATCTCCCGGTTT GAT | |
| 71 | 23S_10B | ACCCCCAGCCACAAGTCATCCGCTAATTT TTC | |
| 72 | 23S_11 | GGTTGGTAAGTCGGGATGACCCCCTTGCC GAA | |
| 73 | 23S_11B | TACCCCCGGAGATGAATTCACGAGGCGCT ACC | |
| 74 | 23S_12 | TTGTTTCCCTCTTCACGACGGACGTTAGC ACC | |
| 75 | 23S_12B | TCTCCCGTGATAACATTCTCCGGTATTCG CAG | |
| 76 | 23S_13 | TAAGCCAACATCCTGGCTGTCTGGGCCTT CCC | |
| 77 | 23S_13B | CCACTTAACCATGACTTTGGGACCTTAGC TGG | |
| 78 | 23S_14 | CATGGTTTAGCCCCGTTACATCTTCCGCG CAG | |
| 79 | 23S_14B | CCAGTGAGCTATTACGCTTTCTTTAAATG ATG | |
| 80 | 23S_15 | ATGCCTCACAGCACACCTTCGCAGGCTTA CAG | |

(continued)

| SEQ No. | Sequence Name | Sequence Information (5'→3') | Target |
|---|---|---|---|
| 81 | 23S_15B | TACCCAACAACGCATAAGCGTCGCTGCCGCAG | |
| 82 | 23S_16 | AACCCTTGGTCTTCCGGCGAGCGGGCTTTTCA | |
| 83 | 23S_16B | CGTTACTTATGTCAGCATTCGCACTTCTGATA | |
| 84 | 23S_17 | TATTAACCTGTTTCCCATCGACTACGCCTTTC | |
| 85 | 23S_17B | TAGGGGTCGACTCACCCTGCCCCGATTAACGT | |
| 86 | 23S_18 | TACACGCTTAAACCGGGACAACCGTCGCCCGG | |
| 87 | 23S_18B | CTTCTCCGTCCCCCCTTCGCAGTAACACCAAG | |
| 88 | 23S_19 | AGGGCATTTGTTGCTTCAGCACCGTAGTGCCT | |
| 89 | 23S_19B | CCTCAGCCTTGATTTTCCGGATTTGCCTGGAA | |
| 90 | 23S_20 | TTGGTATTCTCTACCTGACCACCTGTGTCGGT | |
| 91 | 23S_20B | ATTTGATGTTACCTGATGCTTAGAGGCTTTTC | |
| 92 | 23S_21 | CTCACCTACATATCAGCGTGCCTTCTCCCGAA | |
| 93 | 23S_21B | CATTTTGCCTAGTTCCTTCACCCGAGTTCTCT | |
| 94 | 23S_22 | GTGTTTGCACAGTGCTGTGTTTTTAATAAACA | |
| 95 | 23S_22B | CTGGTATCTTCGACTGATTTCAGCTCCATCCG | |
| 96 | 23S_23 | CGATCAAGAGCTTCGCTTGCGCTAACCCCATC | |
| 97 | 23S_23B | CGGCACCGGGCAGGCGTCACACCGTATACGTC | |

(continued)

| SEQ No. | Sequence Name | Sequence Information (5'→3') | Target |
|---|---|---|---|
| 98 | 23S_24 | TTCGTGCAGGTCGGAACTTACCCGACAAG GAA | |
| 99 | 23S_24B | TAGGACCGTTATAGTTACGGCCGCCGTTT ACC | |
| 100 | 23S_25 | TTGCCGCGGGTACACTGCATCTTCACAGC GAG | |
| 101 | 23S_25B | TGAGTCTCGGGTGGAGACAGCCTGGCCAT CAT | |
| 102 | 23S_26 | TCAAAGCCTCCCACCTATCCTACACATCA AGG | |
| 103 | 23S_26B | GTGTCAAGCTATAGTAAAGGTTCACGGGG TCT | |
| 104 | 23S_27 | CGGATTACGGGTCAACGTTAGAACATCAA ACA | |
| 105 | 23S_27B | TATTTCAAGGTCGGCTCCATGCAGACTGG CGT | |
| 106 | 23S_28 | TTAGCCAACCTTCGTGCTCCTCCGTTACT CTT | |
| 107 | 23S_28B | CGCCCCAGTCAAACTACCCACCAGACACT GTC | |
| 108 | 23S_29 | GCTTTCGCACCTGCTCGCGCCGTCACGCT CGC | |
| 109 | 23S_29B | CTTATGCCATTGCACTAACCTCCTGATGT CCG | |
| 110 | 23S_30 | GTATCAGCCTGTTATCCCCGGAGTACCTT TTA | |
| 111 | 23S_30B | ATGGCCCTTCCATTCAGAACCACCGGATC ACT | |
| 112 | 23S_31 | CCTTGGGACCTACTTCAGCCCCAGGATGT GAT | |
| 113 | 23S_31B | GAGGTGCCAAACACCGCCGTCGATATGAA CTC | |
| 114 | 23S_32 | GCCCACGGCAGATAGGGACCGAACTGTCT CAC | |

(continued)

| SEQ No. | Sequence Name | Sequence Information (5'→3') | Target |
|---|---|---|---|
| 115 | 23S_32B | CCCAGCTCGCGTACCACTTTAAATGGCGAACA | |
| 116 | 23S_33 | CATTGGCATGACAACCCGAACACCAGTGATGC | |
| 117 | 23S_33B | TCCTCTCGTACTAGGAGCAGCCCCCCTCAGTT | |
| 118 | 23S_34 | AGGGAGAACTCATCTCGGGGCAAGTTTCGTGC | |
| 119 | 23S_34B | CAGCACTTATCTCTTCCGCATTTAGCTACCGG | |
| 120 | 23S_35 | TAGCTCAACGCATCGCTGCGCTTACACACCCG | |
| 121 | 23S_35B | TCGTCGTCTTCAACGTTCCTTCAGGACCCTTA | |
| 122 | 23S_36B | AAGGTTAAGCCTCACGGTTCATTAGTACCGGT | |
| 123 | ArOP_5S_1 | CGGCATGGGGTCAGGTGGGACCACCGCGCTAC | 5S rRNA |
| 124 | ArOP_5S_2 | CACACTACCATCGGCGCTACGGCGTTTCACTTC | |
| 125 | ArOP_5S_3 | ATGCCTGGCAGTTCCCTACTCTCGCATGGGGA | |

First, the rRNA removal process was initiated using the total RNA of the target microorganism for analysis, which was extracted through the above-described process, as a sample according to the composition shown in Table 2 below.

[Table 2]

| Composition | Amount |
|---|---|
| Total RNA | 2 μg X 24 tubes |
| 10X DNase□buffer | 1.5 μl X 24 tubes |
| DNase□ | 1 μg (2 U) X 24 tubes |
| Nuclease-free water | Up to 15 μl X 24 tubes |
| Total | 15 μl X 24 tubes |

The reaction solution was prepared with the above composition and reacted at 37°C for 10 minutes in a thermocycler to remove genomic DNA using DNase I. Then, 15 μl of thermostable RNaseH complement buffer (90 mM Tris-HC, 200 mM KCl) was added to each reaction solution, followed by reaction at 75°C for 10 minutes to inactivate DNase I. Then, the temperature was lowered to 25°C, and 2 μl of 23S and 16S rRNA complementary sequences (10 pmol each, 108 types, total 1080 pmol), 0.5 $\mu$l of 5S rRNA complementary sequences (12.5 pmol each, 3 types, total 37.5 pmol), and 1 $\mu$l of 100 mM MgCl$_2$ were added to each tube.

The reaction solution was then heated to 90°C to denature the RNA secondary structure for 1 second and lowered to 65°C, and 2 $\mu$l (10 U) of thermostable RNaseH was added and reacted for 20 minutes to remove rRNA. The reaction products were then denatured again at 90°C for 1 second, followed by an additional rRNA removal reaction at 65°C for 10 minutes. Next, using an RNA purification kit, the reaction products from four tubes were purified into a total of six columns, with one column per tube, and eluted using 40 $\mu$l of nuclease-free water for each column. Here, in the purification process, only RNA of 200 nt or more was selected, enriched, and purified by adjusting the ethanol usage ratio as described in the manual, thereby removing rRNA fragments and tRNA.

After removing rRNA, DNase I reaction was performed to remove rRNA complementary DNA as shown in Table 3 below.

[Table 3]

| Composition | Amount |
|---|---|
| rRNA-depleted RNA | 10 $\mu$l X 24 tubes |
| 10X DNase□buffer | 5 $\mu$l X 24 tubes |
| DNase□ | 5 $\mu$l (10 U) X 24 tubes |
| Nuclease-free water | 30 $\mu$l X 24 tubes |
| Total | 50 $\mu$l X 24 tubes |

The reaction solution was prepared with the above composition and reacted at 25°C, 30°C, 35°C, 40°C, and 45°C for 5 minutes each, and the temperature increase rate was set to 0.1°C/sec for each temperature change to remove rRNA complementary DNA. After the reaction was completed, all 24 tube reaction products were purified using an RNA purification kit with one column, eluted with 20 $\mu$l of nuclease-free water, and only RNA of 200 nt or more was selected and purified again to remove rRNA fragments, tRNA, and rRNA complementary DNA that were not removed. The concentration of purified mRNA was measured using Qubit BR RNA.

To determine whether rRNA was removed before and after the mRNA removal process, microfluidic-based electrophoresis was performed using the Bioanalyzer RNA 6000 pico kit to analyze the size distribution of the mRNA samples.

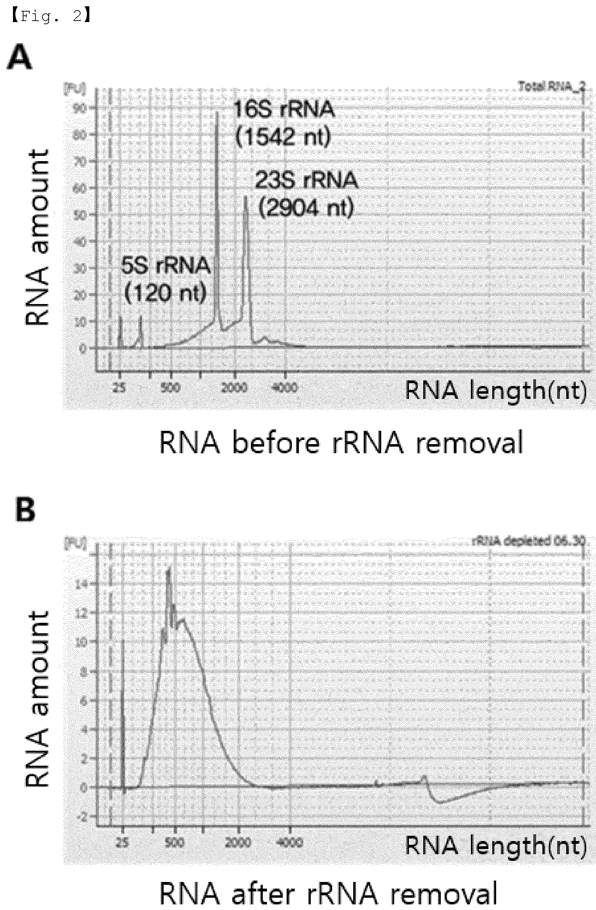

As shown in FIG. 2, the total RNA sample clearly revealed the 5S rRNA (~120 nt), 16S rRNA (~1,500 nt), and 23S rRNA (~2,900 nt) peaks, characteristic of bacteria, (FIG. 2, A), whereas the sample obtained after the rRNA removal detected RNA over a wide length region with no characteristic peaks (FIG. 2, B). This means that rRNA is removed from total RNA with high efficiency, providing a high-quality starting material for generating a library capable of targeting the entire genome.

**Example 2. Process of generating sgRNA library employing total mRNA**

**2-1. mRNA fragmentation**

Using the total mRNA generated in Example 1 as a starting material, the sgRNA library was generated through a series of enzymatic reactions.

First, total mRNA was fragmented into pieces of about 100 nt in length by reacting with Mg$^{2+}$ at high temperature for a short time, to thereby expose the PAM complementary sequence at the 5' end, thereby enabling selection and enrichment and facilitating the subsequent library production process. The reaction was performed with the constitution shown in Table 4 below.

[Table 4]

| Composition | Amount |
|---|---|
| Enriched mRNA | 500 ng |
| 10X RNase fragmentation buffer | 2 $\mu$l |
| Nuclease-free water | Up to 20 $\mu$l |
| Total | 20 $\mu$l |

The reaction solution was prepared with the above composition, followed by fragmentation using a thermocycler at 94°C for 4 minutes and 30 seconds. Immediately after the reaction, the fragmentation was stopped by cooling on ice for 3 minutes, adding 2 µl of stop solution, and reacting at room temperature for 1 minute. The reaction products were then purified with an RNA purification kit, eluted with 10 µl nuclease-free water, and quantified using Qubit BR RNA.

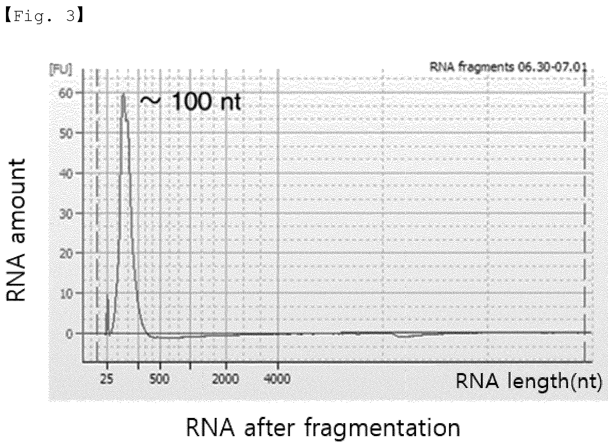

As shown in FIG. 3, the samples before and after mRNA fragmentation were analyzed using the Bioanalyzer RNA 6000 pico kit, and as a result, a peak near 100 nt was observed, confirming that the mRNA was fragmented to a uniform size.

### 2-2. SMART cDNA synthesis

After fragmentation, cDNA synthesis was performed through Switching mechanism at the 5' end of the RNA transcript (SMART). Through the above process, the sample was converted into DNA form, and at the same time, the 1st and 2nd adapters were attached to both ends to function as PCR primers and restriction enzyme recognition sites in the subsequent process. The sequences of the 1st adapter-binding DNA primer (SMART_RT_R) and the 2nd adapter-binding DNA-RNA chimeric primer (SMART_RT_F) used at this time are shown in Table 5 below, and the reaction was initiated with the composition as shown in Table 6 below.

[Table 5]

| Name | Sequence Information (5'→ 3') |
|---|---|
| SMART_RT_R (SEQ ID NO: 1) | AGGTATCTCGAGTCCC**CAGCAG**CNNNNNNN<br>(Bold sequences indicate the EcoP15I recognition site.) |
| SMART_RT_F (SEQ ID NO: 2) | AGTGGTATCAACGCAGAGT***GGCC***AAGCrGrGrG<br>(Bold italicized sequences indicate partial SfiI recognition site.)<br>(rG means RNA G) |

[Table 6]

| Composition | Amount |
|---|---|
| mRNA fragments | 50~200 ng |
| 100 µM SMART_RT_R | 0.25 µl |
| 25 µM dNTP (each) | 0.4 µl |
| Total | 8.13 µl |

The reaction solution was prepared with the above composition, followed by RNA secondary structure denaturation at 72°C for 10 minutes, and cooling on ice for 3 minutes. Then, additional reagents were added with the composition shown in Table 7 below and the reaction was performed at 25°C for 10 minutes.

[Table 7]

| Composition | Amount |
|---|---|
| Denatured RNA (Table 6) | 8.13 µl |
| 5X Superscript II Buffer | 4 µl |
| 100 mM DTT | 2 µl |
| 5M Betaine | 4 µl |
| 1M MgCl$_2$ | 0.12 µl |
| RNase inhibitor murine | 0.5 µl |
| Superscript II reverse transcriptase | 1 µl |
| Total | 19.75 µl |

After that, SMART _RT_F was added, reverse transcription was performed at 42°C for 90 minutes, followed by reaction at 75°C for 10 minutes to inactivate the enzyme. For this purpose, the reaction was performed with the composition in Table 8 below.

[Table 8]

| Composition | Amount |
|---|---|
| Denatured RNA (Table 6) | 19.75 μl |
| 100 μM SMART_RT_F | 0.25 μl |
| Total | 20 μl |

When reacted at 42°C according to the composition above, 7 random base sequences at the 3' end of SMART_RT_R, the 1st adapter-binding primer, were ligated to the mRNA fragment and acted as primers, and reverse transcription by reverse transcriptase proceeded. After reverse transcription, the activity of the reverse transcriptase added a CCC overhang to the 3' end of the cDNA. When RNA GGG at the 3' end of SMART_RT_F, the 2nd adapter-binding primer, is ligated to this overhang site, the template for cDNA synthesis was replaced with SMART _RT_F, allowing reverse transcription to continue, resulting in the attachment of the 1st and 2nd adapters to both ends of the cDNA.

After reverse transcriptase inactivation, RNaseH and RNaseI were added and reacted at 37°C for 30 minutes to degrade the RNA used as a template for reverse transcription. For this purpose, the reaction was performed with the composition in Table 9 below.

[Table 9]

| Composition | Amount |
|---|---|
| cDNA (Table 8) | 20 μl |
| RNaseH | 0.5 μl |
| RNaseI | 0.5 μl |
| Total | 21 μl |

After RNA digestion, cDNA was purified using the ProNex Size-selective purification 3X protocol and eluted with 25 μl of elution buffer (10 mM Tris-HCl, pH 8.5).

**2-3. PCR I**

The PCR I was performed to amplify the sample using the cDNA synthesized in Example 2-2 as a template and the 1st and 2nd adapters as primer sites. The primers used are as shown in Table 10 below, and the reaction was performed with the composition as shown in Table 11 below.

[Table 10]

| Name | Sequence Information (5'→ 3') |
|---|---|
| SMART PCR F(SEQ ID NO: 126) | AGTGGTATCAACGCAGAGTGGCC |
| SMART PCR R(SEQ ID NO: 127) | AGGTATCTCGAGTCCCCAGCAGC |

[Table 11]

| Composition | Amount |
|---|---|
| 5X Q5 reaction buffer | 20 μl X 10 tubes |
| 2.5 mM dNTP (each) | 8 μl X 10 tubes |
| 10 μM SMART_PCR_F | 5 μl X 10 tubes |
| 10 μM SMART_PCR_R | 5 μl X 10 tubes |
| cDNA | 2 μl X 10 tubes |

(continued)

| Composition | Amount |
|---|---|
| Q5 polymerase | 1 µl X 10 tubes |
| Nuclease-free water | 59 µl X10 tubes |
| Total | 100 µl X 10 tubes |

PCR was performed for a total of 12 cycles with a primer annealing temperature of 72°C, an elongation step of 12 seconds, and a final elongation step of 12 seconds, followed by purification using a PCR purification kit and elution with 51 µl of elution buffer (10 mM Tris-HCl, pH 8.5).

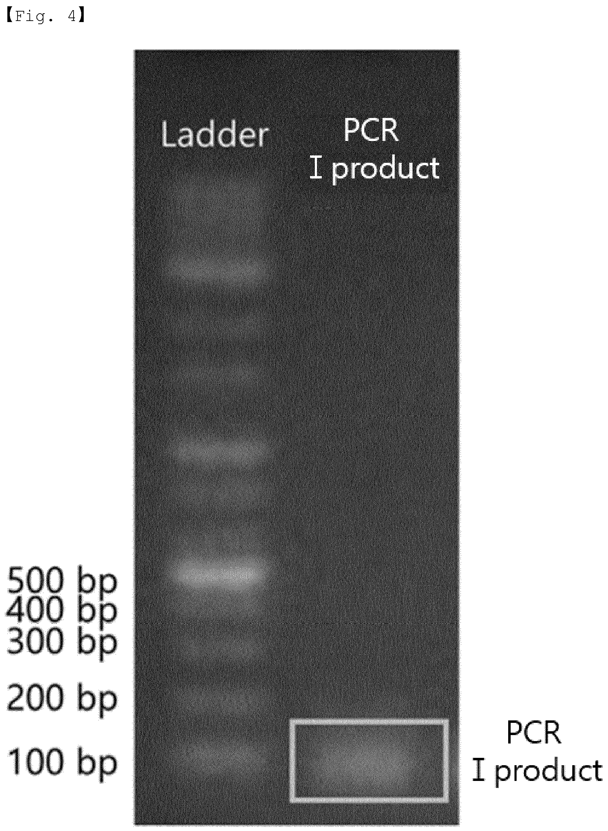

Then, as shown in FIG. 4, electrophoretic analysis confirmed the bands of the PCR product around 100 nt, verifying that the cDNA synthesis, attachment of the 1st and 2nd adapters, and the PCR I were normally performed.

## 2-4. Sfil digestion

The PCR I product has the 2nd adapter attached thereto, wherein the 2nd adapter is designed so that the restriction enzyme Sfi I recognition site contains:

5'...GGCCNNNN/NGGCC...3'
3'...CCGGN/NNNNCCGG...5'
the middle part as follows (see SMART _RT_F in bold in Table 5):
5'...GGCCAAGC/GG 3'
3'...CCGGT/TCGCC 5'

Here, when CC, which is a complementary sequence to the spCas9 PAM sequence, exists at the 5' end of the mRNA used as a template during cDNA synthesis, a complete SfiI recognition site is completed as shown below and cleavage is achieved; whereas CC does not exist, cleavage does not occur:

5'...GGCCAAGC/GG**CC** 3'
3'...CCGGT/TCGCC**GG** 5'

A reaction solution was prepared with the composition shown in Table 12 below, and a restriction enzymatic reaction was performed at 50°C for 120 minutes.

[Table 12]

| Composition | Amount |
|---|---|
| PCR I product | 1 µg X 4 tubes |
| 10x CutSmart buffer | 5 µl X 4 tubes |
| Sfil | 1 µl X 4 tubes |
| Nuclease-free water | Up to 50 µl X 4 tubes |
| Total | 50 µl X 4 tubes |

The reaction product was purified utilizing the ProNex Size-selective purification 3X protocol and eluted with 20 X 4 tubes of elution buffer (10 mM Tris-HCl, pH 8.5) to yield a total of 80 µl of purified product.

## 2-5. 3rd adapter ligation and DNA selection and enrichment containing PAM complementary sequence

After SfiI digestion, the product contains a mixture of cleaved DNA due to the presence of CC, the complementary sequence of spCas9 PAM, at the 5' end of the template mRNA during cDNA synthesis, and uncleaved DNA that remains due to the absence of CC. Here, when a 3rd adapter, a double-stranded DNA with a complementary overhang capable of binding to the overhang of the DNA cleaved by SfiI, is added and the ligation reaction is performed, only DNA containing the PAM complementary sequence is ligated.

First, two single-stranded DNA sequences were complementarily joined to produce a 3rd adapter, which is a double-stranded DNA with an overhang. The sequence of the DNA used is as shown in Table 13 below, and the reaction was performed with the composition as shown in Table 14 below.

[Table 13]

| Name | Sequence Information (5'→ 3') |
|------|-------------------------------|
| Adapter3_F (SEQ ID NO: 5) | Biotin-AGGCTCACTGCATATGCTGAAGT**CAGCA**GC (Underlined sequences indicate AcuI recognition site) (Bold sequences indicate EcoP15I recognition site) |
| Adapter3_R(SEQ ID NO: 8) | GCTGACTTCAGCATATGCAGTGAGCCT |

[Table 14]

| Composition | Amount |
|-------------|--------|
| 10 μM Adapter3_F | 10 μl |
| 10 μM Adapter3_R | 10 μl |
| Nuclease-free water | 180 μl |
| Total | 200 μl |

The reaction solution was prepared with the above composition, heated at 95°C for 5 minutes using a thermocycler to denature the DNA secondary structure, and then cooled up to 25°C at a rate of 0.1°C/sec to generate a double-stranded 3rd adapter.

Then, ligation between the 3rd adapter and the SfiI reaction product was performed with the composition shown in Table 15 below.

[Table 15]

| Composition | Amount |
|-------------|--------|
| SfiI digested product | 150 ng X 6 tubes |
| 500 nM the 3rd adapter | 1 μl X 6 tubes |
| 2X Quick ligase buffer | 10 μl X 6 tubes |
| Quick ligase | 1 μl X 6 tubes |
| Total | 20 μl X 6 tubes |

The reaction solution was prepared with the above composition and ligation was performed at 25°C for 15 minutes.

After ligation, only the DNA containing the PAM complementary sequence (CC) was ligated to the 3rd adapter. Here, biotin was attached to the 3rd adapter, and the strong specific binding between biotin and streptavidin could enable selection and enrichment of only DNA containing the PAM complementary sequence. DNA containing the PAM complementary sequence was bound to the magnetic beads with streptavidin attached to the surface, and the supernatant was removed to remove DNA not containing the PAM complementary sequence. The magnetic beads were resuspended in 72 μl of nuclease-free water.

**2-6. PCR II**

The PCR II was performed to amplify the sample using the DNA containing the PAM complementary sequence bound to the magnetic beads in Examples 2-5 as a template and the 1st and 3rd adapters as primer sites. The primers used are as shown in Table 16 below, and the reaction was performed with the composition as shown in Table 17 below.

[Table 16]

| Name | Sequence Information (5'→ 3') |
|------|-------------------------------|
| SMART PCR R (SEQ ID NO: 127) | AGGTATCTCGAGTCCCCAGCAGC |
| Adapter3_PCR_F (SEQ ID NO: 128) | AGGCTCACTGCATATGCTGAAGTCAGCA GCG |

[Table 17]

| Composition | Amount |
|---|---|
| 5X Q5 reaction buffer | 20 μl X 10 tubes |
| 2.5 mM dNTP (each) | 8 μl X 10 tubes |
| 10 μM Adapter3_PCR_F | 5 μl X 10 tubes |
| 10 μM SMART_PCR_R | 5 μl X 10 tubes |
| DNA attached to streptavidin magnetic beads | 7 μl X 10 tubes |
| Q5 polymerase | 1 μl X 10 tubes |
| Nuclease-free water | 54 μl X 10 tubes |
| Total | 100 μl X 10 tubes |

PCR was performed for a total of 12 cycles with a primer annealing temperature of 72°C, an elongation step of 12 seconds, and a final elongation step of 12 seconds. Then, the supernatant was collected using a magnetic stand, purified using a PCR purification kit, and eluted with 51 μl of elution buffer (10 mM Tris-HCl, pH 8.5).

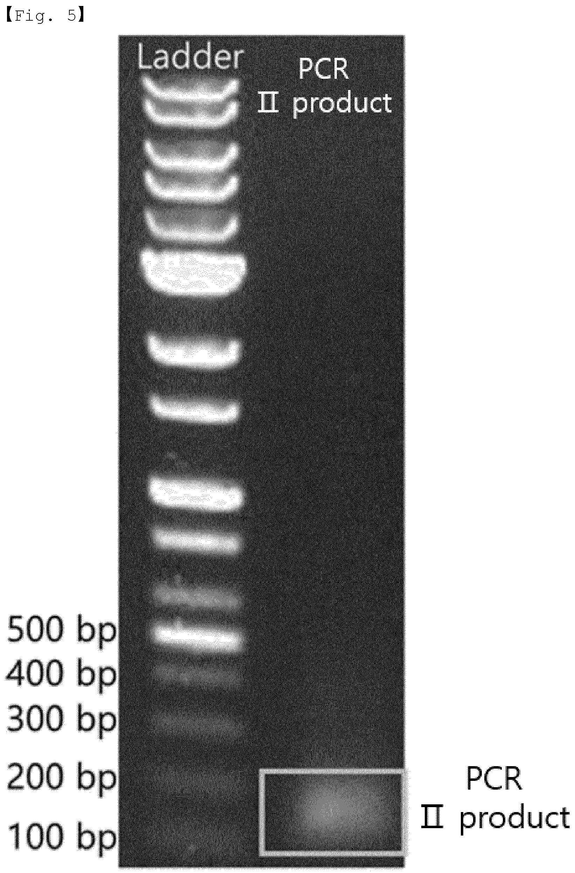

Subsequently, as shown in FIG. 5, electrophoretic analysis confirmed the band of the PCR product around 100 nt, confirming that the SfiI digestion, attachment of the 3rd adapter, and selection and enrichment of DNA containing the PAM complementary sequence were normally performed.

### 2-7. Generation of restriction enzyme-based sgRNA spacer sequence

The PCR II product generated by Examples 2-6 has the 1st and 3rd adapters attached thereto. As shown in Table 5 and Table 13, the 1st and 3rd adapters contain a recognition site for EcoP15I, a type III restriction enzyme, and the 3rd adapter contains a recognition site for AcuI, a type IIS restriction enzyme. These enzymes have recognition sites and cleavage sites that are far apart from each other, resulting in the cleavage of mRNA-derived random sequences located at a defined distance from the adapter sequence. First, the EcoP15I reaction was performed as shown in Table 18 below.

The recognition site of EcoP15I is as follows:

5'...CAGCAG(N)$_{25}$/... 3'
3'...GTCGTC(N)$_{27}$/... 5'. Thus, when cleavage is performed, the following is generated:
5'AGGCTCACTGCATATGCTGAAGTCAGCAGC (N)$_{24}$ 3'
3'TCCGAGTGACGTATACGACTTCAGTCGTCG(N)$_{26}$ 5'.

[Table 18]

| Composition | Amount |
|---|---|
| PCR II product | 1 μl X 4 tubes |
| 10x CutSmart buffer | 4 μl X 4 tubes |
| EcoP15I | 1 μl X 4 tubes |
| 10X ATP | 4 μl X 4 tubes |
| Nuclease-free water | Up to 40 μl X 4 tubes |
| Total | 40 μl X 4 tubes |

The reaction solution was prepared with the above composition and reacted at 37°C for 120 minutes using a thermocycler. Then, the AcuI reaction was performed as shown in Table 19.

The recognition site of AcuI is as follows:

5'...CTGAAG(N)$_{16}$/... 3'
3'...GACTTC(N)$_{14}$/... 5'. Thus, when cleavage is performed, the following is generated:

5'NNNNNNNNNNNNNNNNNN 3'

3'NNNNNNNNNNNNNNNNNNNN 5'.

As shown above, 16nt and 20nt DNA complementarily bind to produce a 20 bp double-stranded DNA product with 2nt overhangs on both sides. The 20nt strand is utilized as the spacer sequence of sgRNA.

[Table 19]

| Composition | Amount |
|---|---|
| EcoP15I reaction product | 40 µl X 4 tubes |
| 10x CutSmart buffer | 1×4 µl tubes |
| AcuI | 2×4 µl tubes |
| 4 mM S-adenosylmethionine | 0.5 µl X 4 tubes |
| Nuclease-free water | 6.5 µl X 4 tubes |
| Total | 50 µl X 4 tubes |

The reaction solution was prepared with the above composition and reacted at 37°C for 120 minutes using a thermocycler. Then, the reaction product was electrophoresed on a 20% (19:1 acrylamide:bis-acrylamide) polyacrylamide gel, where a band of about 20 bp was confirmed and cleaved, and DNA was extracted using a PAGE purification kit, as shown in FIG. 5. The concentration of purified 20 bp long sgRNA spacer DNA was measured via Qubit 1x dsDNA HS.

**2-8. Cloning adapter ligation, 3rd PCR, and Gibson assembly**

The 20 bp long sgRNA spacer DNA generated in Examples 2-7 was ligated with a promoter sequence adapter (4th adapter) and a sgRNA scaffold sequence adapter (5th adapter) for expression, respectively. Here, the sequence transcribed by the promoter is a 20 nt strand, which is the same sequence as the complementary sequence of the mRNA and the template strand of the genomic DNA, and thus the transcribed sgRNA binds to the non-template strand.

Prior to this, two single-stranded DNA sequences were complementarily ligated to prepare double-stranded DNA, the 4th and 5th adapters, respectively. The sequence of the DNA used is as shown in Table 20 below, and the reaction was performed with the same composition reaction conditions as shown in Table 14 below.

[Table 20]

| Name | Sequence Information (5'→ 3') |
|---|---|
| Adapter4_F (SEQ ID NO: 11) | GATCTTTGACAGCTAGCTCAGTCCTAGGTATAA TACTAGT |
| Adapter4_R (SEQ ID NO: 12) | NNACTAGTATTATACCTAGGACTGAGCTAGCTG TCAAAGATC |
| Adapter5_F (SEQ ID NO: 13) | GTTTTAGAGCTAGAAATAGCAAGTTAAAATAAG GCTAGTC |
| Adapter5_R (SEQ ID NO: 14) | GACTAGCCTTATTTTAACTTGCTATTTCTAGCT CTAAAACNN |

Then, ligation of the 4th and 5th adapters and sgRNA spacer DNA was performed with the composition shown in Table 21 below.

[Table 21]

| Composition | Amount |
|---|---|
| sgRNA spacer DNA | 0.1 pmol or more |
| 500 nM the 4th adapter | 5 eq/mol of sgRNA spacer DNA |
| 500 nM the 5th adapter | 5 eq/mol of sgRNA spacer DNA |

(continued)

| Composition | Amount |
|---|---|
| 10X T4 ligase buffer | 2 μl |
| T4 ligase | 1 μl |
| Nuclease-free water | Up to 20 μl |
| Total | 20 μl |

The reaction solution was prepared with the above composition and reacted at 25°C for 120 minutes. The 3rd PCR was then performed using the ligation reaction as a template. Here, the primers used are as shown in Table 22 below, and the reaction was performed with the composition as shown in Table 23 below.

[Table 22]

| Name | Sequence Information (5'→ 3') |
|---|---|
| Adapter4_PCR_F (SEQ ID NO: 129) | GATCTTTGACAGCTAGCTCAGTCCTAGG |
| Adapter5_PCR_R (SEQ ID NO: 130) | GACTAGCCTTATTTTAACTTGCTATTTCTAGCTC |

[Table 23]

| Composition | Amount |
|---|---|
| 5X Q5 reaction buffer | 20 μl |
| 2.5 mM dNTP (each) | 8 μl |
| 10 μM Adapter4_PCR_F | 5 μl |
| 10 μM Adapter5_PCR_R | 5 μl |
| Cloning adapter ligation product | 20 μl |
| Q5 polymerase | 1 μl |
| Nuclease-free water | 41 μl |
| Total | 100 μl |

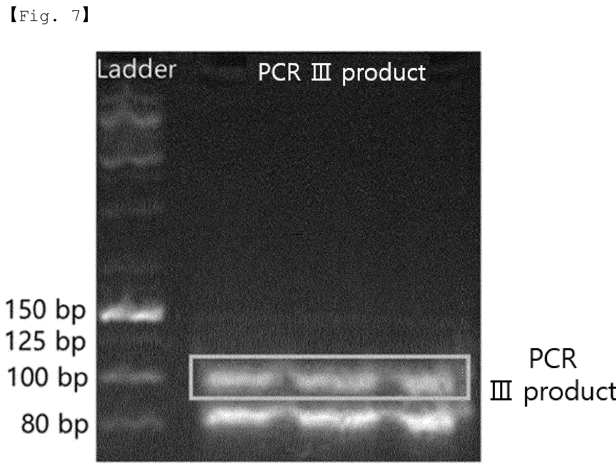

PCR was performed for a total of 20 cycles with a primer annealing temperature of 67°C, an elongation step of 12 seconds, and a final elongation step of 12 seconds. Then, electrophoresis was performed on a 5% agarose gel, confirming that ligation and the 3rd PCR proceeded successfully, as shown in FIG. 7, and a product of 100 bp was isolated by gel extraction.

PCR was then performed on the portion of the pgRNA-bacteria plasmid excluding the sgRNA spacer sequence for Gibson assembly of the 3rd PCR product. The primers used are as shown in Table 24 below, and the reaction was performed with the composition as shown in Table 25 below.

[Table 24]

| Name | Sequence Information (5'→ 3') |
|---|---|
| pgRNA-bacteria_PCR_F (SEQ ID NO: 131) | GTTTTAGAGCTAGAAATAGCAAGTTAAAATAAGGC |
| pgRNA-bacteria_PCR_R (SEQ ID NO: 132) | ACTAGTATTATACCTAGGACTGAGCTAGCTG |

[Table 25]

| Composition | Amount |
|---|---|
| 5X Q5 reaction buffer | 20 $\mu$l |
| 2.5 mM dNTP (each) | 8 $\mu$l |
| 10 $\mu$M pgRNA-bacteria_PCR_F | 5 $\mu$l |
| 10 $\mu$M pgRNA-bacteria_PCR_R | 5 $\mu$l |
| pgRNA-bacteria (plasmid) | 50 ng |
| Q5 polymerase | 1 $\mu$l |
| Nuclease-free water | Up to 100 $\mu$l |
| Total | 100 $\mu$l |

PCR was performed for a total of 30 cycles with a primer annealing temperature of 66°C, an elongation step of 50 seconds, and a final elongation step of 120 seconds.

Gibson assembly was performed using the above pgRNA-bacteria PCR product as a vector and the 3rd PCR product as an insert, in a 1:5 molar ratio, with the composition shown in Table 26 below.

[Table 26]

| Composition | Amount |
|---|---|
| 2X Hifi DNA assembly mix | 10 $\mu$l |
| pgRNA-bacteria PCR product | 0.033 pmol |
| the 3rd PCR product | 0.166 pmol |
| Nuclease-free water | Up to 20 $\mu$l |
| Total | 20 $\mu$l |

The reaction solution was prepared with the above composition and reacted at 50°C for 120 minutes. The reaction was performed at a ratio of 0.2 pmol fragments/20 $\mu$l, and the reaction scale was adjusted as needed. Through the above process, an sgRNA library in a plasmid state was obtained.

## Example 3. Characterization of the generated sgRNA library

To analyze the characteristics of the sgRNA library generated through Example 2, *E. coli* DH5$\alpha$ was transformed with the above library. Then, the pgRNA-bacteria plasmids of 40 colonies were extracted individually and subjected to Sanger sequencing to determine the length of the sgRNA spacer sequence, the reference genome alignment ratio, PAM adjacency, and non-template strand targeting.

### 3-1. Analysis of sgRNA spacer sequence length

Only the sequence of the sgRNA spacer that binds to the target gene was isolated by removing the promoter and sgRNA scaffold sequences for transcription from the sequence obtained through Sanger sequencing.

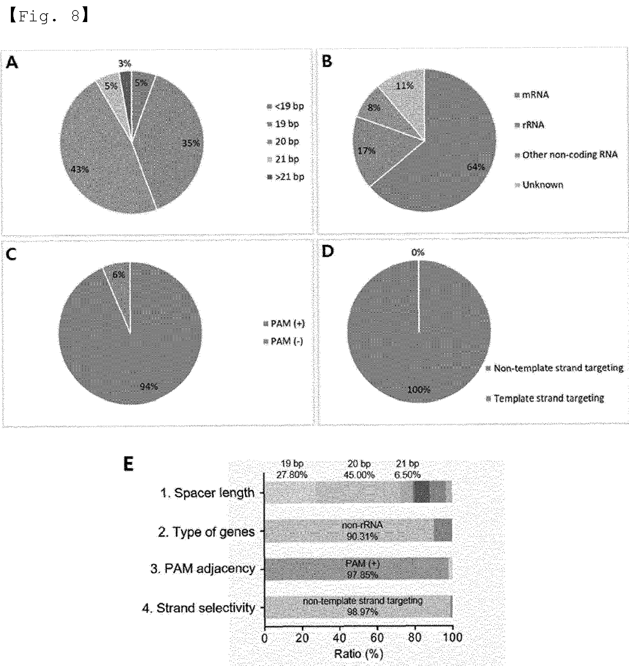

As shown in FIG. 8A, 36 of the 40 sequences (91%) had spacer sequences inserted, of which 2 (5%) were shorter than 19 bp, 14 (35%) were 19 bp, 17 (43%) were 20 bp, 2 (5%) were 21 bp, and 1 (3%) was 21 bp or longer. As a result, it was verified that most (91%) of the sgRNA libraries generated through Example 2 corresponded to the optimal length of 19-21 bp for the spCas9 sgRNA spacer.

### 3-2. Analysis of reference genome alignment

The 36 spacer sequences obtained in Example 2-1 were aligned with the reference genome of *E. coli* K-12 MG1655, which was used to generate the sgRNA library, using BLAST.

As shown in FIG. 8B, 32 of the total (89%) were aligned to the reference genome, of which 26 (72%) were aligned to mRNA and other non-coding RNA genes, and 6 (17%) were aligned to rRNA genes. As a result, it was verified that most of the generated sgRNA libraries were aligned to the reference genome and could be effectively utilized for phenotypic screening.

### 3-3. Analysis of PAM adjacency

Based on the 32 reference genome alignment sequences obtained in Example 2-2, it was analyzed whether the 3' end of the alignment region was adjacent to NGG, which is a PAM sequence of spCas9.

As shown in FIG. 8C, 30 (94%) of the total aligned sequences were adjacent to PAM at the 3' end, and 2 (6%) were not adjacent to PAM. As a result, it was verified that most of the sgRNA sequences aligned to the genome targeted the region adjacent to the PAM, which is essential for the function of Cas9.

### 3-4. Analysis of whether non-template strand targeting occurred

Based on the 32 reference genome alignment sequences obtained in Example 2-2, it was analyzed whether non-template targeting occurred (whether the alignment site was the template strand).

As shown in FIG. 8D, 32 (100%) of the total aligned sequences targeted the non-template strand, and 0 (0%) targeted the template strand. As a result, it was verified that most of the sgRNA sequences aligned to the genome targeted the non-template strand, enabling effective gene repression by dCas9.

### 3-5. Library characterization through next-generation sequencing

In addition to the Sanger sequencing analysis results obtained in Examples 3-1, 3-2, 3-3, and 3-4, next-generation sequencing was performed on the library to more closely analyze the spacer length of the generated sgRNA library, the type of reference genome alignment gene, PAM adjacency, and whether non-template strand targeting occurred.

As shown in FIG. 8E, in view of the spacer length distribution, it was verified that about 80% of sgRNAs corresponded to the optimal length of 19-21 bp for the spCas9 sgRNA spacer. In view of the types of genes aligned to the reference genome, it was confirmed that about 90% were aligned to mRNA and other non-coding RNA. In view of PAM proximity, it was verified that about 98% of sgRNAs targeted regions adjacent to PAM. Finally, it was confirmed that about 99% of sgRNAs targeted the non-template strand of the target gene.

In summary of the above analysis, it is shown that the sgRNA library generated by the present disclosure satisfies the conditions required for sgRNA, such as optimal spacer length, alignment with reference genome, PAM proximity, and non-template strand targeting, and thus can be utilized in various studies including phenotypic screening.

### Example 4. Phenotype-based screening using CRISPRi: Screening for key genes for growth

As an example of utilizing the sgRNA generated by the present disclosure, phenotype-based screening was performed on *E. coli* K-12 MG1655 to select key genes for growth that cause significant growth inhibition when expression is suppressed.

### 4-1. Next-generation sequencing-based library characterization

To obtain a large amount of sgRNA plasmid library to be used for phenotype-based screening, the Gibson assembly reaction performed in Example 2-8 was performed at a 1 ml scale and then purified.

Before selecting key genes for growth, next-generation sequencing was performed using Illumina's Miseq to identify characteristics such as the number of independent sequences in the library and the degree of genomic targeting of the library.

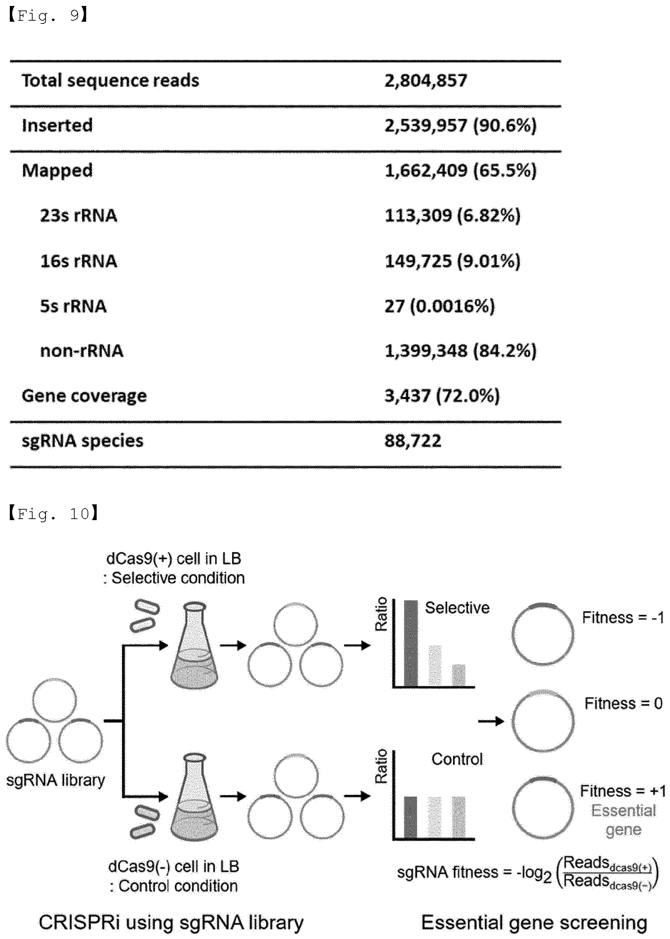

As shown in FIG. 9, the number of different independent sequences in the library was found to be 88,722. Among the total reads, 65.5% of the reads were aligned to the reference genome, and 84.2% of the aligned sequences were aligned to mRNA and other non-coding RNA genes. The proportion of genes aligned by at least one read among the genes in the genome was 72.0%. As a result, it was confirmed that the sgRNA library generated by the present disclosure has a size of approximately 89,000 and can target most of the genes in the genome.

### 4-2. Screening for key genes for growth

A phenotype-based screening experiment was conducted based on the sgRNA library whose characteristics were confirmed in Example 4-1. Since the number of different sequences in the library is about 89,000, transformation was attempted at a scale of 1X or more (89,000 transformants) to ensure that all of these sequences could be included in the screening experiment.

Electroporation-based transformation was performed on MG1655 with plasmid pCD185 expressing dCas9 and MG1655 with plasmid pCD185 NC with dCas9 deleted, at 1.17X and 2.35X relative to library size, respectively. The transformed cells were added in S.O.C. (1:4, V/V) and allowed to recover at 37°C for 1 hour, then washed with fresh LB (1:1, V/V), inoculated into LB to a final volume of 100 ml, and cultured until the $OD_{600}$ of live cells reached approximately 1.0.

Here, the LBs contained chloramphenicol, an antibiotic marker for pCD185 plasmid, and carbenicillin, an antibiotic marker for the pgRNA-bacteria plasmid, at concentrations of 34 $\mu$g/ml and 100 $\mu$g/ml, respectively. Then, 5 ml of each culture solution was taken, and the plasmid was extracted, followed by next-generation sequencing as shown in the schematic diagram in FIG. 10.

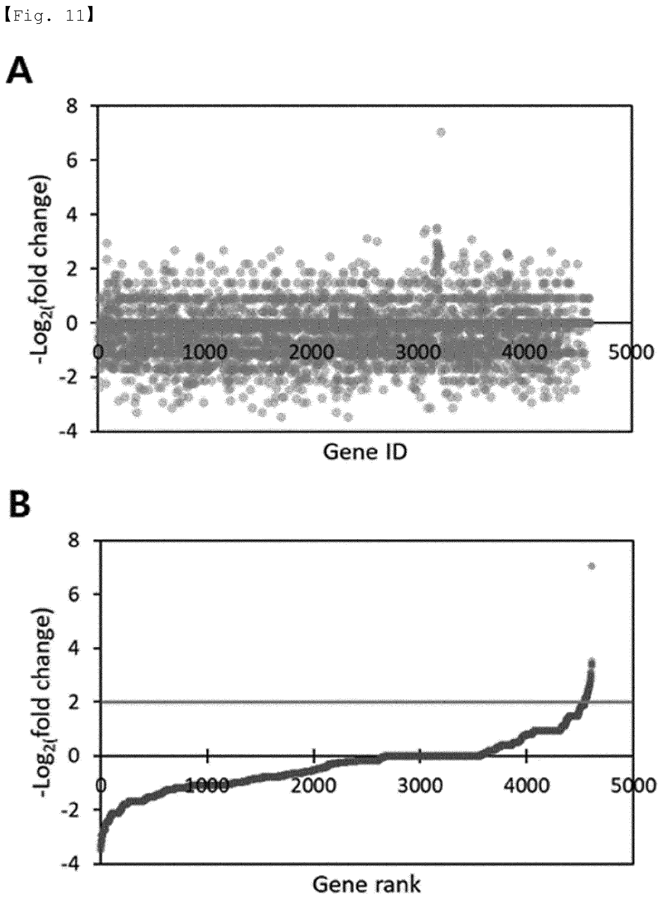

As shown in FIG. 11A, normalization was performed by dividing the number of reads per gene by the total number of reads, and then the ratio of reads assigned to each gene in cells expressing and not expressing dCas9 was compared with each other.

$$\text{gene ratio fold change} = \frac{\frac{gene\ reads\ in\ dCas9}{\sum dCas9\ reads} \times 10^6 + 1}{\frac{gene\ reads\ in\ dCas9NC}{\sum dCas9NC\ reads} \times 10^6 + 1}$$

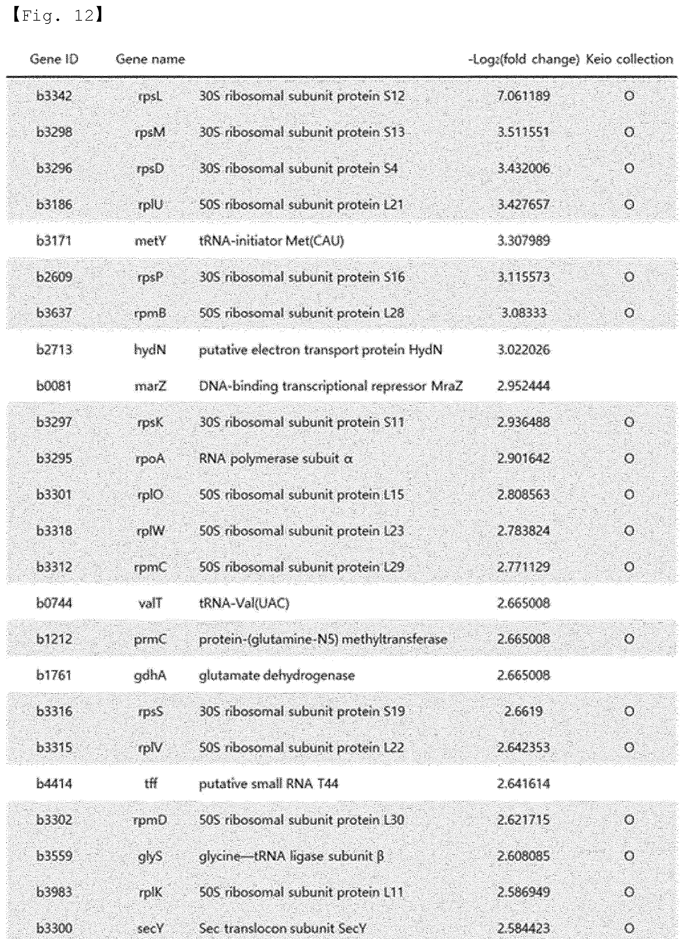

As shown in FIG. 11B and FIG. 12, genes with a 4-fold or greater decrease in abundance in cells expressing dCas9 compared to reads in cells not expressing dCas9 (genes with a $-Log_2$ (fold change) value of 2 or more) were examined to obtain a total of 66 genes, and 36 (55%) of these genes were confirmed to match the Keio collection, one of the previously reported essential gene lists of *E. coli.* As a result, it was confirmed that the sgRNA library generated by the present disclosure can be used to perform phenotype-based screening, such as selection of key genes whose growth is suppressed upon inhibition.

## Claims

1. A primer set for generating an sgRNA library, comprising:
   a 1st adapter-binding primer that acts as a reverse transcription primer using fragmented mRNA of a target organism as a template and binds a 1st adapter to the 5' end of cDNA; and a 2nd adapter-binding primer that acts as a template strand extension primer of a reverse transcription product and binds a 2nd adapter to the 3' end of cDNA, wherein

   (1) the 1st adapter-binding primer has a structure of the following General Formula I-1;

   5'-X-Y-Z-3'                (I-1)

   wherein X is a site that functions as a primer for PCR I amplification, comprising 9 to 14 bases; Y is a type III restriction enzyme recognition site, comprising 6 bases; and Z is a random heptamer site having a hybridization sequence complementary to the fragmented mRNA of the target organism, comprising 7 bases; and
   (2) the 2nd adapter-binding primer is a DNA-RNA chimeric primer, and has a structure of the following General Formula I-2;

   5'-X'-Y'-Z'-3'                (I-2)

   wherein X' is a binding site of the primer in PCR I amplification, comprising 16 to 20 bases; Y' is a partial recognition site of any one of restriction enzymes SfiI, BamHI, or KpnI, comprising 8 bases; and Z' is a site that extends a strand (template) through switching mechanism at 5' end of RNA template (SMART), as an RNA sequence that hybridizes complementarily with an overhang at 3' end of the reverse transcription product, comprising three guanine (G) bases;
   wherein the 2nd adapter-binding primer employs as a template, the reverse transcription product, which is obtained after performing reverse transcription using the 1st adapter-binding primer;
   the 1st adapter acts as a type III restriction enzyme recognition site; and
   the 2nd adapter acts as a partial recognition site of any one of restriction enzymes SfiI, BamHI, and KpnI.

2. A primer set for generating an sgRNA library, comprising:
a 3rd adapter-generating forward primer (A3F) and a 3rd adapter-generating reverse primer (A3R) that are hybridized with a restriction enzyme cleavage product generated using the primer set of claim 1, followed by ligation, wherein

(1) the 3rd adapter-generating forward primer (A3F) has a structure of the following General Formula II-1;

5'-biotin-X''-Y''-Z''-I-3'          (II-1)

wherein X" is a binding site of the primer in PCR II amplification; Y'' is a type IIS restriction enzyme recognition site, comprising 6 bases; Z" is a type III restriction enzyme recognition site, comprising 6 bases; and I is a site having a hybridization sequence complementary to the restriction enzyme cleavage product, comprising 1 to 4 bases; and
(2) the 3rd adapter-generating reverse primer (A3R) has a structure of the following General Formula II-2;

5'-Z'''-Y'''-X'''-3'          (II-2)

wherein Z''' is a site where a type III restriction enzyme recognition site is generated by binding to the restriction enzyme cleavage product; Y''' is a type IIS restriction enzyme recognition site, comprising 6 bases; and X''' is a binding site of the primer in PCR II amplification;
the 3rd adapter is a double-stranded DNA to which biotin is attached, having the structure of the following General Formula II-3;

5'-biotin-X*-Y*-Z*-3'          (II-3)

wherein X* is a type IIS restriction enzyme recognition site for cleavage between the 3rd adapter and the restriction enzyme cleavage product generated using the primer set of claim 1; Y* is a type III restriction enzyme recognition site; and Z* is a site for ligation with the restriction enzyme cleavage product generated using the primer set of claim 1; and
the 3rd adapter acts as a label site for separating a protospacer adjacent motif (PAM) adjacent sequence.

3. A primer set for generating an sgRNA library, comprising:
a 4th adapter-generating forward primer (A4F); a 4th adapter-generating reverse primer (A4R); a 5th adapter-generating forward primer (A5F); and a 5th adapter-generating reverse primer (A5R), which are ligated to both ends of the DNA produced by sequentially cleaving the double-stranded DNA product to which the 3rd adapter is ligated using the primer set of claim 2, wherein

(1) the 4th adapter-generating forward primer (A4F) comprises a promoter sequence for transcription of an sgRNA spacer and 15 to 40 bases for performing Gibson assembly with an sgRNA-expressing vector;
(2) the 4th adapter-generating reverse primer (A4R) has a structure of the following III-1;

5'-A-B-3'          (III-1)

wherein A is a site having a complementary hybridization sequence comprising 2 bases with an overhang at the 5' end of a single-stranded DNA comprising 20 bases; and B is a site having a hybridization sequence complementary to the 4th adapter-generating forward primer (A4F);
(3) the 5th adapter-generating forward primer (A5F) comprises an sgRNA scaffold sequence and 15 to 40 bases for performing Gibson assembly with the sgRNA-expressing vector;
(4) the 5th adapter-generating reverse primer (A5R) has a structure of the following III-2;

5'-A'-B'-3'          (III-2)

wherein A' is a site having a hybridization sequence complementary to the 5th adapter-generating forward primer (A5F); and B' is a site having a complementary hybridization sequence comprising 2 bases with an overhang of the 3' end of a single-stranded DNA comprising 20 bases;
the 4th adapter acts as a promoter site for sgRNA expression; and

the 5th adapter has an sgRNA scaffold sequence.

4. The primer set of claim 2 or 3, wherein the ligation is performed by one ligation agent selected from the group consisting of SplintR ligase, bacteriophage T4 ligase, *E. coli* ligase, Afu ligase, Taq ligase, Tfl ligase, Mth ligase, Tth ligase, Tth HB8 ligase, Thermus species AK16D ligase, Ape ligase, LigTk ligase, Aae ligase, Rm ligase, Pfu ligase, ribozyme, and variants thereof.

5. The primer set of claim 1, wherein the 1st adapter-binding primer comprises SEQ ID NO: 1.

6. The primer set of claim 1, wherein the 2nd adapter-binding primer comprises SEQ ID NO: 2, 3 and 4.

7. The primer set of claim 2, wherein the 3rd adapter-generating forward primer (A3F) comprises any one selected from SEQ ID NOs: 5, 6, and 7, and the 3rd adapter-generating reverse primer (A3R) comprises any one selected from SEQ ID NOs: 8, 9, and 10.

8. The primer set of claim 3, wherein the 4th adapter-generating forward primer (A4F) comprises SEQ ID NO: 11, and the 4th adapter-generating reverse primer (A4R) comprises SEQ ID NO: 12.

9. The primer set of claim 3, wherein the 5th adapter-generating forward primer (A5F) comprises SEQ ID NO: 13, and the 5th adapter-generating reverse primer (A5R) comprises SEQ ID NO: 14.

10. A method for generating an sgRNA library comprising:

   (a) fragmenting mRNA of a target organism;
   (b) synthesizing cDNA, using the fragmented mRNA as a template, comprising the 1st adapter of claim 1 that acts as a reverse transcription PCR primer binding site at the 5' end and the 2nd adapter of claim 1 that acts as a recognition site of any one restriction enzyme selected from SfiI, BamHI, and KpnI at the 3' end;
   (c) obtaining a restriction enzyme treatment product by treatment with any one restriction enzyme selected from SfiI, BamHI, and KpnI;
   (d) ligating the 3rd adapter of claim 2 that forms a complementary hybridization with an overhang at the 3' end of the restriction enzyme treatment product of Step (c) and acts as a labeling site for separating a protospacer adjacent motif (PAM) adjacent sequence;
   (e) selecting only cDNA to which the 3rd adapter is ligated;
   (f) treating with a type III restriction enzyme; and
   (g) producing sgRNA spacer DNA by treatment with type II S restriction enzyme.

11. The method of claim 10, wherein the sgRNA spacer DNA is produced by the complementary binding of a single-stranded DNA comprising 20 or 22 bases and a single-stranded DNA comprising 16 or 18 bases.

12. The method of claim 10, wherein the fragmenting of the mRNA in Step (a) comprises:

   i) extracting total RNA of the target organism;
   ii) generating an rRNA-DNA double strand by treatment with a DNA probe having a sequence complementary to rRNA; and
   iii) removing rRNA by treatment with an RNase that selectively degrades only rRNA of the rRNA-DNA double strand of Step ii).

13. The method of claim 12, wherein the RNase is a thermostable RNaseH.

14. The method of claim 10, wherein the fragmenting of the mRNA is performed by any one selected from the group consisting of physical fragmentation, enzyme-based fragmentation, and chemical fragmentation.

15. The method of claim 10, wherein the selecting of the cDNA to which the 3rd adapter is ligated is performed using biotin-streptavidin binding.

16. The method of claim 10, further comprising:

   (h) ligating the 4th adapter of claim 3, which acts as a PCR promoter, and the 5th adapter of claim 3, which acts as an sgRNA scaffold, to both ends of the sgRNA spacer DNA; and
(i) producing an sgRNA library in a plasmid state, comprising DNA excluding the sgRNA spacer sequence as a vector and DNA containing the sgRNA spacer sequence as an insert.

17. The method of claim 16, wherein the sgRNA is bound to a non-template strand.

18. A kit for phenotype-based screening comprising the sgRNA library produced by the method of claim 16.

19. A phenotype-based screening method using the sgRNA library produced by the method of claim 16.

【Fig. 1a】

【Fig. 1b】

**sgRNA plasmid library**

【Fig. 2】

**A**

RNA before rRNA removal

**B**

RNA after rRNA removal

【Fig. 3】

RNA after fragmentation

【Fig. 4】

【Fig. 5】

【Fig. 6】

sgRNA
Spacer sequence

【Fig. 7】

【Fig. 8】

【Fig. 9】

| | |
|---|---|
| Total sequence reads | 2,804,857 |
| Inserted | 2,539,957 (90.6%) |
| Mapped | 1,662,409 (65.5%) |
| 23s rRNA | 113,309 (6.82%) |
| 16s rRNA | 149,725 (9.01%) |
| 5s rRNA | 27 (0.0016%) |
| non-rRNA | 1,399,348 (84.2%) |
| Gene coverage | 3,437 (72.0%) |
| sgRNA species | 88,722 |

【Fig. 10】

CRISPRi using sgRNA library          Essential gene screening

【Fig. 11】

【Fig. 12】

| Gene ID | Gene name | | -Log₂(fold change) | Keio collection |
|---------|-----------|--|---|---|
| b3342 | rpsL | 30S ribosomal subunit protein S12 | 7.061189 | O |
| b3298 | rpsM | 30S ribosomal subunit protein S13 | 3.511551 | O |
| b3296 | rpsD | 30S ribosomal subunit protein S4 | 3.432006 | O |
| b3186 | rplU | 50S ribosomal subunit protein L21 | 3.427657 | O |
| b3171 | metY | tRNA-initiator Met(CAU) | 3.307989 | |
| b2609 | rpsP | 30S ribosomal subunit protein S16 | 3.115573 | O |
| b3637 | rpmB | 50S ribosomal subunit protein L28 | 3.08333 | O |
| b2713 | hydN | putative electron transport protein HydN | 3.022026 | |
| b0081 | mraZ | DNA-binding transcriptional repressor MraZ | 2.952444 | |
| b3297 | rpsK | 30S ribosomal subunit protein S11 | 2.936488 | O |
| b3295 | rpoA | RNA polymerase subuit α | 2.901642 | O |
| b3301 | rplO | 50S ribosomal subunit protein L15 | 2.808563 | O |
| b3318 | rplW | 50S ribosomal subunit protein L23 | 2.783824 | O |
| b3312 | rpmC | 50S ribosomal subunit protein L29 | 2.771129 | O |
| b0744 | valT | tRNA-Val(UAC) | 2.665008 | |
| b1212 | prmC | protein-(glutamine-N5) methyltransferase | 2.665008 | O |
| b1761 | gdhA | glutamate dehydrogenase | 2.665008 | |
| b3316 | rpsS | 30S ribosomal subunit protein S19 | 2.6619 | O |
| b3315 | rplV | 50S ribosomal subunit protein L22 | 2.642353 | O |
| b4414 | tff | putative small RNA T44 | 2.641614 | |
| b3302 | rpmD | 50S ribosomal subunit protein L30 | 2.621715 | O |
| b3559 | glyS | glycine—tRNA ligase subunit β | 2.608085 | O |
| b3983 | rplK | 50S ribosomal subunit protein L11 | 2.586949 | O |
| b3300 | secY | Sec translocon subunit SecY | 2.584423 | O |

## INTERNATIONAL SEARCH REPORT

| International application No. |
|---|
| **PCT/KR2023/014843** |

| **A.** | **CLASSIFICATION OF SUBJECT MATTER** |
|---|---|

**C12N 15/10**(2006.01)i; **C12N 15/113**(2010.01)i; **C12Q 1/6806**(2018.01)i; **C40B 50/06**(2006.01)i

According to International Patent Classification (IPC) or to both national classification and IPC

| **B.** | **FIELDS SEARCHED** |
|---|---|

Minimum documentation searched (classification system followed by classification symbols)

C12N 15/10(2006.01); C12N 15/113(2010.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: sgRNA, 라이브러리(library), 어댑터(adapter), 프라이머(primer), mRNA, 제한효소(restriction enzyme), cDNA, 역전사(reverse transcription)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X<br>Y | ARAKAWA, H. et al. A method to convert mRNA into a gRNA library for CRISPR/Cas9 editing of any organism. Sci. Adv. 24 August 2016, vol. 2, thesis no. e1600699, pp. 1-10.<br>　See abstract; pages 1, 3 and 7-9; and figures 1-2. | 1,5-6<br>2-4,7-19 |
| Y | CRAVERO, K. et al. Biotinylated amplicon sequencing: A method for preserving DNA samples of limited quantity. Practical Laboratory Medicine. 2018, vol. 12, thesis no. e00108, pp. 1-10.<br>　See abstract; and figure 1. | 2-4,7-19 |
| Y | BALDWIN, A. et al. An Easy, Cost-Effective, and Scalable Method to Deplete Human Ribosomal RNA for RNA-seq. Current Protocols. 2021, vol. 1, thesis no. e176, pp. 1-13.<br>　See abstract; and figure 1. | 12-13 |
| A | KR 10-2017-0020091 A (MEDICINAL BIOCONVERGENCE RESEARCH CENTER) 22 February 2017 (2017-02-22)<br>　See entire document. | 1-19 |

✓ Further documents are listed in the continuation of Box C.　　　✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 January 2024** | **09 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

# EP 4 596 691 A1

## INTERNATIONAL SEARCH REPORT

<table>
<tr><td colspan="2">International application No.</td></tr>
<tr><td colspan="2">**PCT/KR2023/014843**</td></tr>
</table>

| C. | DOCUMENTS CONSIDERED TO BE RELEVANT | | |
|---|---|---|
| Category* | Citation of document, with indication, where appropriate, of the relevant passages | Relevant to claim No. |
| A | US 2018-0051278 A1 (TWIST BIOSCIENCE CORPORATION) 22 February 2018 (2018-02-22) See entire document. | 1-19 |

Form PCT/ISA/210 (second sheet) (July 2022)

## INTERNATIONAL SEARCH REPORT

| International application No. |
| --- |
| **PCT/KR2023/014843** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

 a.   ☑ forming part of the international application as filed.

 b.   ☐ furnished subsequent to the international filing date for the purposes of international search (Rule 13*ter*.1(a)),

  ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/KR2023/014843**

| Patent document cited in search report | | | Publication date (day/month/year) | Patent family member(s) | | | Publication date (day/month/year) |
|---|---|---|---|---|---|---|---|
| KR | 10-2017-0020091 | A | 22 February 2017 | None | | | |
| US | 2018-0051278 | A1 | 22 February 2018 | AU | 2017-315294 | A1 | 21 March 2019 |
| | | | | CA | 3034769 | A1 | 01 March 2018 |
| | | | | CN | 109996876 | A | 09 July 2019 |
| | | | | EA | 201990487 | A1 | 30 August 2019 |
| | | | | EP | 3500672 | A1 | 26 June 2019 |
| | | | | EP | 3500672 | A4 | 20 May 2020 |
| | | | | GB | 2568444 | A | 15 May 2019 |
| | | | | IL | 264966 | A | 31 October 2021 |
| | | | | IL | 264966 | B | 31 October 2021 |
| | | | | JP | 2019-528070 | A | 10 October 2019 |
| | | | | JP | 6854340 | B2 | 07 April 2021 |
| | | | | KR | 10-2019-0041000 | A | 19 April 2019 |
| | | | | KR | 10-2212257 | B1 | 04 February 2021 |
| | | | | SG | 11201901563 | A | 28 March 2019 |
| | | | | US | 10053688 | B2 | 21 August 2018 |
| | | | | US | 10975372 | B2 | 13 April 2021 |
| | | | | US | 2018-0312834 | A1 | 01 November 2018 |
| | | | | US | 2021-0180046 | A1 | 17 June 2021 |
| | | | | WO | 2018-038772 | A1 | 01 March 2018 |

Form PCT/ISA/210 (patent family annex) (July 2022)